# EUROPEAN PATENT APPLICATION

(11) **EP 1 985 318 A1**
(43) Date of publication of application: **29.10.2008**
(21) Application number: 07706330.3
(22) Date of filing: 13.02.2007
(51) Int. Cl.: A61L 27/00, G01N 30/88, C12M 3/00

(54) **PROCESS FOR PRODUCING BONE GRAFTING MATERIAL, BONE GRAFTING MATERIAL, THREE-DIMENSIONAL SUPPORT FOR CELL CULTURE, AND SEPARATION SUPPORT FOR CHROMATOGRAPHY**

(30) Priority: 14.02.2006 JP 2006037301
(71) Applicant: The University of Tokyo, Bunkyo-ku, Tokyo 113-8654 (JP); National Institute of Advanced Industrial Science and Technology, Tokyo 100-8921 (JP); Next21 K.K., Tokyo 113-0033 (JP); Gauss K.K., Aioi-shi, Hyogo 678-0092 (JP)
(72) Inventor: TEI, Yuichi, Tokyo 113-8654 (JP); TERAOKA, Kay, Nagoya-shi, Aichi 463-8560 (JP); SUZUKI, Shigeki, Tokyo 113-0033 (JP); SHIMIZU, Kotaro, Tokyo 113-0033 (JP); TAKANE, Katsuhisa, Aioi-shi, Hyogo 678-0071 (JP)
(74) Representative: Pitchford, James Edward
(86) International application number: PCT/JP2007/000082
(87) International publication number: WO 2007/094134

(57) **Abstract**

A novel method for producing a bone filling material is provided. The method comprises the steps of: (a) kneading ingredient comprising calcium-based material and material comprising binder; (b) molding a predetermined shape of the mixture obtained in step (a) with an injection molding machine having a mold; (c) removing the binder contained in the mold formed in step (b) (i.e., degreasing) to obtain a degreased body; (d) and heating and sintering the degreased body obtained in step (c) to obtain a sintered body.

## Description

### BACKGROUND OF THE INVENTION

### Field of the Invention

The present invention relates to a bone filling material which is produced applying the powder injection molding method, and a method for producing the bone filling material and a three-dimensional cell culture carrier. In particular, the present invention relates to a method for producing a minute bone filling material having a plurality of protruding parts, a bone filling material, and a three-dimensional cell culture carrier.

### Description of the Related Art

In the conventional therapeutic technique, when a part of bone tissue is lost in an accident or in a surgical treatment, regeneration of the lost bone was promoted, for example, by filling the lost part of bone with bone filling material which replaces the lost bone tissue. Calcium phosphate-based material such as hydroxyapatite and β-TCP, or a biodegradable plastic such as polylactic acid was, for example, used as ingredients of the bone filling material.

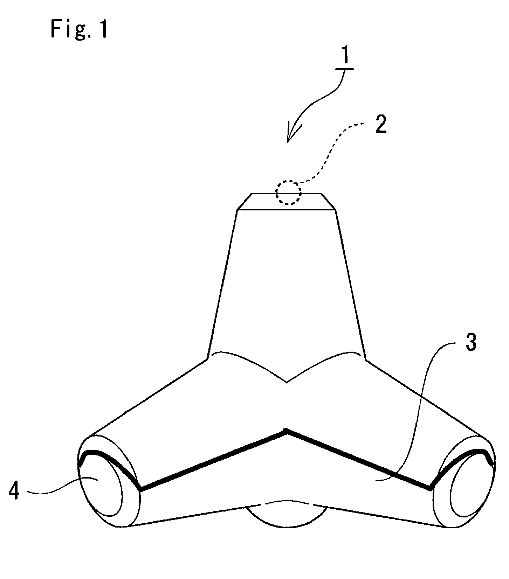

A tetrapod-shaped bone filling material (bone fixing material) is disclosed in Japanese Patent Laid-Open No. 2004-97259 (e.g., Fig. 1 to Fig. 3 of the bulletin). Note that "tetrapod" is a trademark. And a method of melt molding using a split mold is disclosed (in paragraph [0014] and [0025] of the bulletin) as a method for producing tetrapod^{™}-shaped bone filling material. However, the bone filling material produced by the method of melt molding using a split mold does not always have enough strength. And when calcium phosphate-based material and biodegradable polymers were produced by the melt molding, there is also a problem that the dimensional accuracy and the molding density of a resultant bone filling material become uneven. Uneven surface condition of the bone filling material is another problem. In addition, the production method of the bone filling material disclosed in the bulletin is not suitable for mass production.

A method for producing a rod-shaped or sheet-shaped bone filling material using polylactic acid or hydroxyapatite is disclosed in Japanese Patent Laid-Open No. 11-206871 (paragraph [0018]), wherein an injection molded bone filling material is uniaxially extended. However the resultant bone filling material is a polymer molecule such as polylactic acid or a mixture of the polymer molecule and calcium phosphate-based material. And the embodiment of the bulletin discloses the production of a sheet-formed bone filling material by extrusion molding (paragraph [0023]), but a complex-shaped (e.g., tetrapod-shaped) bone filling material cannot be produced by extrusion molding.

Also, the powder injection molding method was used to produce metal parts and ceramics. However, in the powder injection molding method, material with poor biocompatibility is used as binder. So there was no idea of molding a material which is embedded in vivo for the purpose of tissue replacement, by the powder injection molding method. Besides the bone filling material has to have bone tissue replacement ability in vivo. So when a bone filling material is molded from powders such as calcium-based materials by the powder injection molding method, a preferred bone filling material cannot be obtained.

The object of the present invention is to provide a novel method for producing bone filling material.

The object of the present invention is to provide a method for producing bone filling material with less variation in shape and density.

The object of the present invention is to provide a method for producing bone filling material which is easy to be removed from a mold when it is injection molded.

The object of the present invention is to provide bone filling material having a predetermined strength and a method for producing the same.

The object of the present invention is to provide a bone filling material having a predetermined pharmacological effect and a method for producing the same.

Cell culture has been so far performed two-dimensionally. In contrast, the object of the present invention is to provide a carrier which can perform cell culture three-dimensionally.

The object of the present invention is to provide usages of the above mentioned bone filling material which have not been considered so far. In particular, usages of a three-dimensional cell culture carrier, a separating carrier for chromatography, and the like is provided.

### SUMMARY OF THE INVENTION

A bone filling material with a complicated minute shape cannot be produced by the ordinary powder injection molding method, because, for example, the molded body adheres to a fixed mold. This invention is made preferably for the purpose of mass production of the bone filling material having preferred physical properties. The present invention is based on the following idea. That is, a bone filling material having physical properties, such as less variation in surface area and density, and appropriate level of hardness as a bone filling material, can be produced by applying the powder injection molding method, which has been used basically for manufacturing metal parts or ceramics, to a production method of a bone filling material using calcium-based material such as calcium phosphate-based material as ingredient powders ingeniously. Since each of the bone filling material has even surface area and density, appropriate dosage of pharmaceutical agent can be administered, even when the pharmaceutical agent is incorporated in the bone filling material. In addition, since each of the bone filling material is uniform in size, the whole of bone filling materials can obtain porosity while maintaining the intensity of each bone filling material. The present invention is also based on the following idea. The resultant molded body can be prevented from adhering to a fixed mold by using a mold and materials of the present inveniton.

The method for producing a bone filling material of the present invention basically comprises the steps of: (a) kneading ingredient comprising calcium-based material and material comprising binder; (b) molding a molded body having a predetermined shape from a kneaded material obtained in step (a) with an injection molding machine having a mold; (c) removing the binder contained in the molded body (i.e., degreasing) to obtain a degreased body, the molded body being obtained in step (b); and (d) heating and sintering the degreased body to obtain a sintered body, the degreased body being obtained in step (c).

A method for producing a bone filling material according to an aspect of the present invention is the above described method, wherein the calcium-based material comprises one or both of calcium phosphate-based material and calcium carbonate-based material, and wherein the calcium phosphate-based material is one or more than one kind of hydroxyl apatite, carbonate apatite, fluorapatite, chlorapatite, β-TCP, α-TCP, calcium metaphosphate, tetra calcium phosphate, calcium hydrogen phosphate, octa calcium phosphate, calcium dihydrogen phosphate, calcium pyrophosphate, the salts thereof, and the solvates thereof. These materials effectively replace with osteocytes. So a bone filling material contributing to bone regeneration can be provided.

The bone filling material obtained by the production method of the present invention is generally minute, and need to have appropriate physical properties as a bone filling material. So a preferred aspect of the production method of the present invention produces a preferred bone filling material by using a certain kind and amount of binder added to ingredients. A specific example of the production method is one of the above described methods for producing a bone filling material, wherein the binder comprises (meta) acrylic-based resin, wax lubricant, and lubricant. Another example of the production method is one of the above described methods for producing a bone filling material, wherein the binder comprises wax lubricant, and wherein the wax lubricant comprises wax having melting point of 40°C to 100°C. Since wax lubricant having low melting point is used, the bone filling material can be taken out of a mold easily, which enables effective production of the bone filling material. A preferred embodiment of the present invention is one of the above described methods for producing a bone filling material wherein the binder comprises (meta) acrylic-based resin, ethylene-vinyl acetate copolymer, paraffin wax, stearic acid and dibutyl phthalate, and wherein the step (a) comprises the steps of: putting the (meta) acrylic-based resin and the ethylene-vinyl acetate copolymer in a kneading machine; putting the ingredient, the paraffin wax, and the stearic acid in the kneading machine while kneading the (meta) acrylic-based resin and the ethylene-vinyl acetate copolymer; and putting the dibutyl phthalate in the kneading machine while kneading the (meta) acrylic-based resin, the ethylene-vinyl acetate copolymer, the paraffin wax, and the stearic acid. Although producing a bone filling material is not easy, the most desirable bone filling material can be produced by using particular resin.

A method for producing a bone filling material according to a certain aspect of the present invention is one of the above described methods for producing a bone filling material wherein the material comprising binder further comprising glass components. If the strength of a bone filling material can be controlled, the application of the bone filling material will be extended. And the bone filling material including glass components can achieve high strength.

A method for producing a bone filling material according to an aspect of the present invention is one of the above described methods for producing a bone filling material wherein the material comprising binder further comprising salt or sugar. The bone filling material of the present invention is to culture cells such as bone cells in vivo or in vitro. So it is desired that the bone filling material have a surface to which cells can be attached easily. And if the bone filling material is produced by using ingredients including salt or sugar, a porous bone filling material can be obtained by removing salt or sugar with water. Then, pores where salts or sugars were embedded appear, and a preferred physical structure for cellular culture can be obtained.

A method for producing a bone filling material according to an aspect of the present invention is one of the above described methods for producing a bone filling material, wherein the bone filling material has a plurality of protruding parts (e.g., more than four protruding parts), and wherein the mold comprises: a fixed mold having an inlet where material (e.g., kneaded material) is injected; a movable mold being contacted with the fixed mold when the material is injected, the movable mold being apart from the fixed mold after a molded body is formed, wherein the inlet of the fixed mold is located at a tip part of one of the protruding parts so that the material for injection molding is injected from the tip part of the protruding part, wherein a parting face of the fixed mold and the movable mold has an inclined surface, the inclined surface inclines toward the inlet of the fixed mold from the edge face to the center of the parting face, and wherein the movable mold has dent parts for providing predetermined wedges to the movable mold. Producing a bone filling material accompanies difficulties. Those who skilled in the art think of producing the protruding parts independently and then combining them together, instead of adopting a method of injection molding. But since the bone filling material of the present invention is minute, it is difficult to produce the protruding parts separately and combine them together. The mold of the present invention can produce an injection body having a plurality of protruding parts in one injection molding operation. The mold of the present invention also made it easy for a molded body to be taken out from the fixed mold, for example, by providing a three-dimensional parting face, wherein the parting face of the fixed mold and the movable mold has an inclined surface toward the inlet from the edge to the center of the parting face. For example, when a split mold having a flat parting face is used to mold a molded body, the molded body is highly likely to be stuck in a fixed mold, and it is difficult to remove the molded body from the fixed mold. But the above described mold having inclined parting face lowered the possibility that a molded body remains in a fixed mold.

A preferred embodiment of the present invention is the above described methods for producing a bone filling material, wherein the height of the wedge is 1 µm to 1x10² µm. If a molded body remains in the fixed mold, bone filling material can not be produced. The wedge part serves as a wedge to the moving part, which prevents a molded body being left in the fixed mold when the mold is opened.

A preferred embodiment of the present invention is one of the above described methods for producing a bone filling material, wherein the height of the wedge is 1 µm to 1x10² µm. If a molded body remains in the fixed mold, bone filling material can not be produced. The groove parts increase the surface area of the movable mold part, thereby preventing a molded body from being left in the fixed mold when the mold is opened.

A preferred embodiment of the present invention is one of the above described methods for producing a bone filling material, wherein the bone filling material has four protruding parts (preferably so-called a tetrapod-shaped bone filling material wherein four protruding parts extend toward each vertex of the regular tetrahedron.), and wherein the movable mold has an ejector pin located inside the protruding part. It is very important for injection molding to remove a molded body from a mold. In this embodiment, a protruding pin is arranged so that it is located inside a protruding part (so that the pin is directed from the other end of the tip of a protruding part through the center of the mold toward the tip of the protruding part.), so a molded body can be taken out effectively.

A preferred embodiment of the present invention is one of the above described methods for manufacturing a bone filling material wherein the bone filling material has a plurality of protruding parts. And the mold comprises: a fixed mold; a movable mold being contacted with the fixed mold when material is injected, the movable mold being apart from the fixed mold after a molded body is formed, wherein an inlet of the material for injection molding is located at a parting face of the fixed mold and the movable mold, the parting face of the fixed mold and the movable mold has an inclined surface, the inclined surface inclines toward the center of the bone filling material from the edge face to the center of the parting face, and the movable mold has groove parts for providing predetermined wedges to the movable mold. An injection body having a plurality of protruding parts can be obtained in one injection molding operation by an ingenious mold at the timing of injection molding.

A method for producing a bone filling material according to an aspect of the present invention is the above described method for producing a bone filling material wherein the step (c) comprises a step of heating-up at 1 °C/hour to 3x10² °C/hour (preferably 10 °C/hour to 2x10² °C/hour) until the temperature reaches a value in the range of 110°C to 300 °C, which is the range of a first maintaining period. A binder removal step, for example, has several stages of heating-up period and maintaining period in accordance with the pyrolysis temperature of resin contained in binder. Since binder is removed through several heating-up stages, pyrolysis can be effectively performed from the resin having low pyrolysis temperature, thereby effectively removing binder. In particular, effective pyrolysis of resin having low pyrolysis temperature improves sintering performance. In the present invention, since temperature is raised as above described, the resin having low pyrolysis temperature can be effectively pyrolyzed.

A method for producing a bone filling material according to an aspect of the present invention is one of the above described methods for producing bone filling material further comprising the step of impregnating or administering pharmaceutical agent to the sintered body obtained in the sintering step. And a preferred embodiment of the method for producing bone filling material of the present invention is one of the above described methods for producing bone filling material whererin the pharmaceutical agent comprises one or more than one kind of an osteogenesis/chondrogenesis promoter (including chondrogenesis promoting factor), a joint disease therapeutic agent, a preventive and/or therapeutic agent for bone/cartilage disease, a bone-regenerating agent, a bone resorption-suppressing substance, an angiogenesis promoter, an antibacterial agent, an antibiotics, or an anticancer agent. The conventional bone filling material was aimed merely at bone regeneration by being administered to defective parts of bone tissues. The bone filling material of the present invention, pharmaceutical agents being administered thereto, can prevent infections of affected sites as well as promote bone regeneration. Namely, the present invention can provide a bone filling material comprising: a plurality of protruding parts, wherein an adhesiveness-imparting agent is impregnated, administered or powder blended on the surface of the protruding parts; and calcium-based materials.

A method for producing a bone filling material according to an aspect of the present invention is one of the above described methods for producing a bone filling material comprising the step of impregnating or administering an adhesiveness-imparting agent to the sintered body obtained in step (d). The adhesiveness-imparting agent, for example, is thrombin. Also, an embodiment of the present invention is one of the above described methods for producing a bone filling material further comprising the steps of: preparing a composition including two kinds of adhesiveness-imparting agents; obtaining a first bone filling material group by impregnating a certain bone filling material group with a first composition or by applying a first composition to a certain bone filling material group; obtaining a second bone filling material group by impregnating a certain bone filling material group with a second composition or by applying a second composition to a certain bone filling material group, wherein the bone filling material groups including the first bone filling material group and the second bone filling material group are used as the bone filling materials, and wherein the first composition comprises: one or more than one kind of compound represented by the below-described general formula (I) or (II), or a first compound including 3 to 8 repeating units represented by the below-described general formula (III); and a first diluent (or a carrier), and wherein the second composition comprises: one or more than one kind of compound represented by the below-described general formula (I) or (II), or a second compound including 3 to 8 repeating units represented by the below-described general formula (III); and a second diluent:

X₁-(OCH₂CH₂)ₙ-X₂ (I)

wherein X₁ and X₂ are the same or different and each represents -R¹COONHS (where R¹ is a C₁₋₇ alkylene group), -COR¹COONHS (where R¹ is a C₁₋₇ alkylene group), -NOCOR¹-R² (where R¹ is a C₁₋₇ alkylene group, and R² is a maleimide group), -R¹NH₂ (where R¹ is a C₁₋₇ alkylene group), -R¹SH (where R¹ is a C₁₋₇ alkylene group), or -CO₂PhNO₂ (where Ph is an o-, m-, or p-phenylene group), and n is an integer of 80 to 1000.

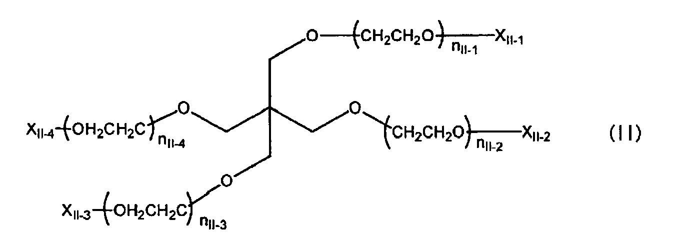

wherein X_{II-1} to X_{II-4} are the same or different and each represents -R¹COONHS (where R¹ is a C₁₋₇ alkylene group), -COR¹COONHS (where R¹ is a C₁₋₇ alkylene group), -NOCOR¹-R² (where R¹ is a C₁₋₇ alkylene group, and R² is a maleimide group), -R¹NH₂ (where R¹ is a C₁₋₇ alkylene group), -R¹SH (where R¹ is a C₁₋₇ alkylene group), or -CO₂PhNO₂ (where Ph is an o-, m-, or p-phenylene group), and n_{II-1} to n_{II-4} are the same or different and each represents an integer of 20 to 250.

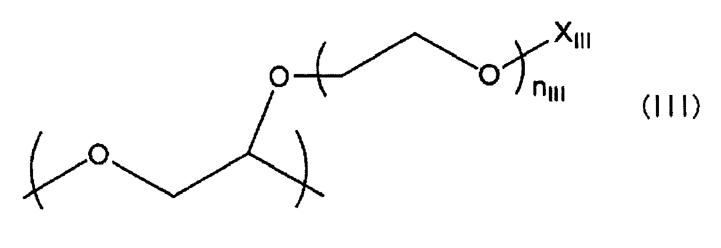

wherein X_{III} represents -R¹COONHS (where R¹ is a C₁₋₇ alkylene group), -COR¹COONHS, -NOCOR¹-R² (where R¹ is a C₁₋₇ alkylene group, and R² is a maleimide group), -R¹NH₂ (where R¹ is a C₁₋₇ alkylene group), -R¹SH (where R¹ is a C₁₋₇ alkylene group), or -CO₂PhNO₂ (where Ph is an o-, m-, or p-phenylene group), and n_{III} represents an integer of 10 to 150.

A more preferred embodiment of the above described method for producing a bone filling material is a method, wherein the first compound comprises one or more than one kind of compound represented by the general formula (I) or (II), wherein X₁, X₂ or X_{II-1} to X_{II-4} are the same or different, and each represents -NOCOR¹-R² or -R¹NH₂, and wherein the second compound comprises one or more than one kind of compound represented by the general formula (I) or (II), wherein X₁, X₂ or X_{II-1} to X_{II-4} are the same or different, and each represents -COR¹COONHS, -R¹SH, or -CO₂PhNO₂ (where Ph is an o-, m-, or p-phenylene group).

A bone filling material is intended to be administered in vivo. And it is desirable that a plurality of the bone filling materials be combined with each other, thereby maintaining a steric structure. The conventional bone filling material has a fragile steric structure which exhibits low-strength. However, the above described adhesiveness-imparting agent enhances the adhesiveness of the each bone filling material and the adhesiveness of the bone filling material to bone tissues, thereby maintaining the steric structure. When a heat-resistant adhesiveness-imparting agent is used, the adhesiveness-imparting agent may be mixed with ingredient powders so that a bone filling material which is powder blended with the adhesiveness-imparting agent may be obtained (in this case, the adhesiveness-imparting agent exists on the surface of the bone filling material, and when the adhesiveness-imparting agent on the surface is replaced with bone tissues, a new surface having the other adhesiveness-imparting agent appears, thereby maintaining the adhesiveness of the bone filling material). Also, a powdered adhesiveness-imparting agent may be sprayed on the surface of a molded body or a sintered body. Furthermore, adhesiveness-imparting agents may be added to the surface of the bone filling material by powder blending, wherein a plurality of bone filling agents and powdered adhesiveness-imparting agents are mixed together and then agitated as needed. Namely, the present invention also provides a bone filling material comprising: a plurality of protruding parts, wherein an adhesiveness-imparting agent is impregnated, administered or powder blended on the surface of the protruding parts; and calcium-based materials.

A method for producing a bone filling material according to an aspect of the present invention is the above described method for producing a bone filling material, wherein the bone filling material has four protruding parts, the four protruding parts extending from the center of the regular tetrahedron form of the bone filling material toward each vertex thereof. A bone filling material having this structure can form preferred continuous holes, so cells and the like can be cultured easily.

A usage example of a bone filling material according to an aspect of the present invention is a three-dimensional cell culture carrier comprising one of the above described bone filling materials including calcium-based material, and plurality of protruding parts. A preferred embodiment of the three-dimensional cell culture carrier comprises a bone filling material, wherein a pharmaceutical agent or an adhesiveness-imparting agent is impregnated, administered or powder blended on the surface of the bone filling material. Conventionally, it was intended that a cell culture was performed in a two-dimensional environment, such as in a Petri dish etc. But the present invention enables an in vitro cell culture to be performed in a three-dimensional environment effectively.

A usage example of a bone filling material according to a certain aspect of the present invention is a separating carrier for chromatography comprising a bone filling material including a plurality of protruding parts and containing calcium-based material. As demonstrated in the example described below, the bone filling material of the present invention has preferred sorbability, and the sorbability can be controlled, for example, by adjusting voids of the bone filling material. So a plurality of bone filling materials can be used as separating carriers for chromatography.

In the present invention, the powder injection molding method, adjusted in accordance with calcium phosphate-based material as ingredient powders, was used for producing the bone filling material. So the present invention can provide a method for producing bone filling material having high form accuracy and less variation in the volume.

In the present invention, since a predetermined wax was used as binder, a method for producing a bone filling material which is easy to be removed from a mold can be provided.

As demonstrated in the example described below, the present invention can provide a bone filling material having a predetermined strength and a method for producing the same.

The bone filling material of the present invention, having been administered a pharmaceutical agent on the surface of a molded body, can act as a pharmaceutical agent having a predetermined pharmacological effect. So the present invention can provide a method for producing a bone filling material having a predetermined pharmacological effect and a method for producing the same.

Cell culture has been so far performed two-dimensionally. But, in contrast, as demonstrated in the example described below, the present invention can provide a carrier which can perform cell culture three-dimensionally by using a plurality of bone filling materials effectively.

The present invention provides usage examples of the above mentioned bone filling material which have not been considered so far. In particular, the usage of a three-dimensional cell culture carrier, a separating carrier for chromatography, and the like can be provided.

### BREIF DESCRIPTION OF THE DRAWINGS

Fig. 1 is a conceptual diagram showing an example of a mold used in the method for producing a bone filling material of the present invention.
Fig. 2 is a conceptual diagram for explaining an example of a tetrapod-shaped bone filling material. Fig. 2(A) is a side view, Fig. 2(B) is a top view, and Fig. 2(C) is a perspective view.
Fig. 3 is a conceptual diagram for explaining an example of a tetrapod-shaped bone filling material with its top portion cut off. Fig. 3(A) and Fig. 3(B) are side views, Fig. 3(C) is a top view, and Fig. 3(D) is a perspective view.
Fig. 4 is a conceptual diagram for explaining an example of a bone filling material having three protruding parts. Fig. 4(A) is a perspective view, and Fig. 4(B) is a top view.
Fig. 5 is a conceptual diagram for explaining an example of a hemispherical bone filling material. Fig. 5(A) is a perspective view, and Fig. 5(B) is a bottom view.
Fig. 6 is a conceptual diagram for explaining an example of a bone filling material having one or a plurality of hollowed parts in the undersurface part. Fig. 6(A) is a perspective view, and Fig. 6(B) is a bottom view.
Fig. 7 is a conceptual diagram for explaining an example of a double-headed-shaped bone filling material. Fig. 7(A) is a perspective view, and Fig. 7(B) and Fig. 7(C) are side views.
Fig. 8 is a conceptual diagram for explaining an example of a bone filling material having protruding parts on the center thereof. Fig. 8(A) is a perspective view, and Fig. 8(B) is a side view.
Fig. 9 is a conceptual diagram for explaining an example of a cross-shaped bone filling material. Fig. 9(A) is a perspective view, Fig. 9(B) is a top view, Fig. 9(C) is a side view, and Fig. 9(D) is a bottom view.
Fig. 10 is a conceptual diagram for explaining an example of a nearly planar bone filling material. Fig. 10(A) is a perspective view, Fig. 10(B) is a top view, and Fig. 10(C) is a side view.
Fig. 11 is a conceptual diagram for explaining an example of a bone filling material having protruding parts on one or both ends of the plane face.
Fig. 12 is a conceptual diagram for explaining an example of a bone filling material having an inclined top surface. Fig. 12(A) is a perspective view, and Fig. 12(B) is a side view.
Fig. 13 is a CAD generated drawing of a bone filling material.
Fig. 14 is a photograph, in place of a diagram, showing a bone filling material obtained in Example 1.
Fig. 15 is an electron microgram, in place of a diagram, showing a bone filling material obtained in Example 1.
Fig. 16 is an electron microgram, in place of a diagram, showing clustered bone filling materials obtained in Example 1.
Fig. 17 shows a relationship between the sintering temperature and the bending strength of the bone filling material obtained in Example 1.
Fig. 18 are CT images by micro X-rays, in place of a diagram, showing gatherings of the bone filling materials obtained in Example 1 and the existing artificial bone product. Fig. 18(A) shows gatherings of the bone filling materials of the present invention. Fig. 18(B) shows gatherings of the conventional artificial bone granules. Fig. 18(C) shows the bone filling materials of the present invention filled in a cryotube. Fig. 18(D) shows the existing artificial bone products filled in a cryotube.

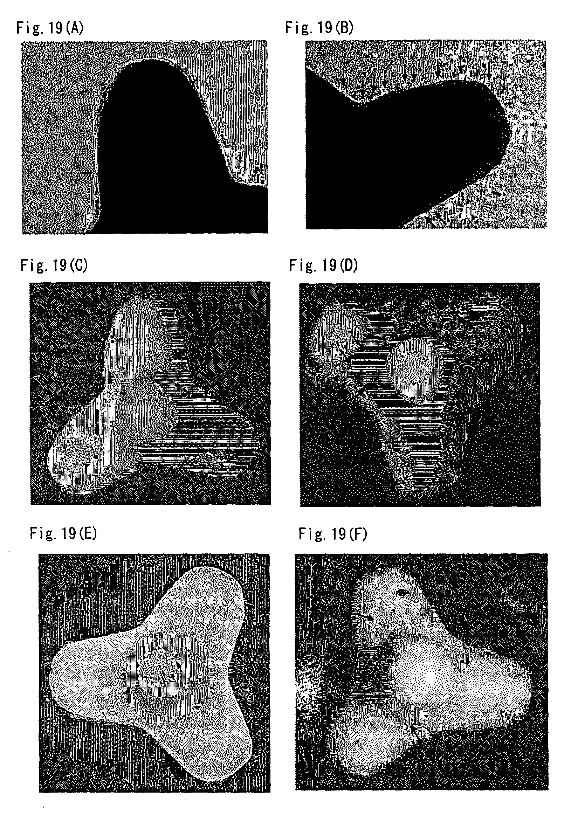

Fig. 19(A) to (F) are photographs, in place of diagrams, examining cellular adhesiveness of a bone filling material of the present invention. Fig. 19 (A) is a photograph showing a bone filling material without cultured cells after four days culture. Fig. 19 (B) is a photograph, after four days culture, showing a bone filling material on which osteoblast-like cell lines MC3T3 were cultured. Fig. 19 (C) is a photograph showing a bone filling material without cultured cells which is Alkaline Phosphatase (ALP) stained after six days culture. Fig. 19 (D) is a photograph, Alkaline Phosphatase (ALP) stained after six days culture, showing a bone filling material on which osteoblast-like cell lines MC3T3 were cultured. Fig. 19 (E) is a photograph showing a bone filling material without cultured cells which is Alkaline Phosphatase (ALP) stained after ten days culture. Fig. 19 (F) is a photograph, Alkaline Phosphatase (ALP) stained after ten days culture, showing a bone filling material on which osteoblast-like cell lines MC3T3 were cultured.

### DESCRIPTION OF THE PREFFERED EMBODIMENTS

Hereinafter, a method for producing a bone filling material of the present invention is explained. The method for producing a bone filling material of the present invention basically comprises the steps of: (a) kneading ingredient comprising calcium-based material and material comprising binder; (b) molding a molded body having a predetermined shape from a kneaded material obtained in step (a) with an injection molding machine having a mold; (c) removing the binder contained in the molded body (i.e., degreasing) to obtain a degreased body, the molded body being obtained in step (b); and (d) heating and sintering the degreased body to obtain a sintered body, the degreased body being obtained in step (c). The method may include publicly known steps such as an after treatment step for a molded body.

Since each of the bone filling material obtained by the production metod of the present invneiton has uniformity in size, appropriate dosage of pharmaceutical agent can be administered, even when the pharmaceutical agent is incorporated in the bone filling material. Furthermore, the bone filling materials, when administered, have appropriate porosity while maintaining the strength of each bone filling material, because the bone filling material has uniform density and its size can be controllable. Each steps of the method for producing a bone filling material is explained in the following.

### Kneading Step

In the kneading step, ingredient comprising calcium-based material and material comprising binder are kneaded. In this step, it is preferred to use powdered ingredient. In this step, powdered ingredient and sub materials such as binders are mixed so that they are made to be suitable for injection molding.

### Kinds of Ingredient Powder

Calcium-based materials, for example, are used as powdered ingredients. Examples of the calcium-based materials include calcium phosphate-based materials, calcium carbonate-based materials, calcium lactate, and calcium gluconate. Among them, calcium phosphate-based material or calcium carbonate-based material is preferred. Specific examples of calcium phosphate-based materials as powdered ingredients include one or more than one kind of hydroxyl apatite, carbonic acid apatite, fluorapatite, chlorapatite, β-TCP, α-TCP, calcium metaphosphate, tetracalcium phosphate, calcium hydrogen phosphate, calcium dihydrogen phosphate, calcium pyrophosphate, the salts thereof, and the solvates and dihydrates thereof. Among them, β-TCP or hydroxyl apatite is preferred. Specific examples of calcium carbonate-based materials include calcium carbonate and calcium hydrogen carbonate. Among them, calcium carbonate is preferred. Note that, the powdered ingredients are not specifically limited to these materials, and well-known materials used as ingredients of the bone filling materials can be used as appropriate.

### Size of the Ingredient Powder

When the size of the ingredient powder is too small, many binders are required for forming a mold, and the physical properties of the resultant bone filling material is deteriorated. On the other hand, when the size of the ingredient powder is too large, there are risks that the ingredient powders get into gaps between a screw and a cylinder of a molding machine, or sintering of ingredient powder does not proceed. In the present invention, powder injection molding is basically performed, but the ingredient powder used in the powder injection molding is not always metal powder. As a result of an experiment, the grain size of the ingredient powder is, for example, from 0.01 µm to 100 µm (both inclusive, same below), and is preferably from 0.1 µm to 20 µm. In a general powder metallurgy, for example, powders with the size of 100µm are used. For example, in Japanese Patent Laid-Open No. 2004-97259, hydroxyl apatite having a grain size of less than 150 µm is used (paragraph [0025]). But in the present invention, it is preferred that ingredient powder having relatively small grain size be used to improve fluidity of kneaded material (mixture of ingredient powders and binders) and improve the density of a sintered body. On the other hand, although the bone filling material of the present invention requires certain strength, it is assumed to be eroded by osteoclasts. From this perspective, the grain size may be from 0.1 µm to 50 µm, and may preferably be from 0.5 µm to 10 µm.

### Sub Materials

In the kneading step, materials such as binder, other than the ingredient, are mixed with the ingredient. The examples of the binder include (meta) acrylic-based resin, wax lubricant, (preferably, thermoplastic resin other than (meta) acrylic-based resin) and material having lubricant. The example of methacrylic-based resin or acrylic-based resin is methacrylate resin or acrylate resin, and it specifically includes a polymer of n-butyl methacrylate or methyl methacrylate, or a copolymer of n-butyl methacrylate and methyl methacrylate. The molecular weight of methacrylic-based resin or the acrylic-based resin is not specifically limited, but it is preferred to be adjusted as appropriate so that the physical properties of the resultant bone filling material are not lost, and the weight average molecular weight is, for example, 1x10³ to 1x10⁵. The content of methacrylic-based resin or acrylic-based resin in the binders is not specifically limited, but it is preferred to be adjusted as appropriate so that the physical properties of the resultant bone filling material are not lost, and the content is, for example, 1% by weight to 50% by weight.

The example of the wax lubricant is wax having a melting point of 40°C to 100°C, and the melting point is preferably 40°C to 70°C. As the wax having the above melting point, for example, well known paraffin wax can be used as appropriate. A molded body can be easily removed from a mold at the time of injection molding by using wax having the above melting point. It is more preferred to use wax having a melting point of 60°C to 65°C, because a molded body can be removed from a mold without cooling the mold too much.

The examples of wax lubricant include one or more than one kind of: hydrocarbon oil such as liquid paraffin, squalene, and squalane; higher fatty acid such as oleic acid, tall oil, and isostearic acid; higher alcohol such as lauryl alcohol, oleyl alcohol, isostearyl alcohol and the octyldodecanol; silicone oil such as methyl polysiloxane, methyl phenyl polysiloxane, methyl hydrogen polysiloxane, and decamethyl polysiloxane; ester such as isopropyl myristate, isopropyl palmitate, hexyl laurate, oleyl oleate, decyl oleate, octyl dodecyl myristate, hexyl decyl dimethyl octanoate, diethyl phthalate, and dibutyl phthalate; animal and plant oil such as avocado oil, camellia oil, turtle oil, macademia nut oil, corn oil, sesame oil, persic oil, wheat germ oil, sasanqua oil, castor oil, linseed oil, safflower oil, cotton oil, perilla oil, Chinese bean oil, peanut oil, tea seed oil, kaya oil, rice bran oil, jojoba oil, apricot kernel oil, olive oil, carrot oil, grape seed oil, rape seed oil, camellia oil, jojoba oil, egg yolk oil, lanolin oil, and mink oil; and glycerine such as glycerine, diglycerol, triglycerine, glycerine trioctanoate, and glycerine triisopalmitate. The melting point of wax lubricant can be controlled by adjusting molecular weight and composition ratio of these raw materials as appropriate.

The molecular weight of wax lubricant is not specifically limited, but it is preferred to be adjusted as appropriate so that the physical properties of the resultant bone filling material are not lost, and the weight average molecular weight is, for example, 1x10² to 1x10⁶. The content of wax lubricant in the binders is not specifically limited, but it is preferred to be adjusted as appropriate so that the physical properties of the resultant bone filling material are not lost, and the content is, for example, 1% by weight to 50% by weight.

The examples of a thermoplastic resin include one or more than one kind of: polyacetal resin, (meta) acryl resin, polyolefin resin, ethylene - vinyl acetate copolymer, and polyvinyl butyral. However, in the present invention, resins other than (meta) acrylate resin and (meta) acrylic - based resin are preferred as thermoplastic resins. Among them, ethylene - vinyl acetate copolymer is preferred.

The molecular weight of a thermoplastic resin is not specifically limited, but it is preferred to be adjusted as appropriate so that the physical properties of the resultant bone filling material are not lost, and the weight average molecular weight is, for example, 1x10³ to 1x10⁵. The content of a thermoplastic resin in the binders is not specifically limited, but it is preferred to be adjusted as appropriate so that the physical properties of the resultant bone filling material are not lost, and the content is, for example, 1% by weight to 50% by weight.

The examples of lubricant (other than wax lubricant) include one or more than one kind of: stearic acid, a salt of stearic acid, a hydrate of stearic acid, a hydrate of a salt of stearic acid, and C₁-C₅ alkyl stearic acid (C₁-C₅ alkyl represents an alkyl group having 1 to 5 carbon atoms, same below); or one of these materials and polyethylene glycol or one of these materials and polyglycerol. The content of lubricant in the binders is not specifically limited, but it is preferred to be adjusted as appropriate so that the physical properties of the resultant bone filling material are not lost, and the content is, for example, 0.5% by weight to 15% by weight. The molded bodies can be removed from a mold easily by using the above lubricant. The lubricant may also act as a dispersing agent.

The phthalic acid ester group is another compound comprising the binders. It is reported that the phthalic acid ester group is harmful to the human body. But in a preferred embodiment of the present invention, the binders are thermally decomposed almost completely. So chemical compounds having poor biocompatibility, such as the phthalic acid ester group, can be contained in binders. The example of the phthalic acid ester group is C₁-C₅ alkyl phthalate such as dibutyl phthalate. As demonstrated in the example described below, a bone filling material having more preferred physical properties could be obtained by consciously using the phthalic acid ester group.

The molecular weight of the phthalic acid ester group is not specifically limited, but it is preferred to be adjusted as appropriate so that the physical properties of the resultant bone filling material are not lost, and the weight average molecular weight is, for example, 1x10⁴ to 1x10⁷. The phthalic acid ester group with poor volatility is also preferred. The content of the phthalic acid ester group in the binders is not specifically limited, but it is preferred to be adjusted as appropriate so that the physical properties of the resultant bone filling material are not lost, and the content is, for example, 0% by weight to 20% by weight, and is preferably 0.5% by weight to 15% by weight.

### Binder Loadings

Binders are removed by being thermally decomposed in the binder removing step described below. The parts where binders existed basically become voids. So the porosity and the intensity of a resultant bone filling material can be adjusted by controlling the quantity of binders added to ingredient. But in general, the amount of binders required is the amount that is enough to fill spaces between particles of ingredients. This is because if the amount of binders is not enough, appropriate fluidity can not be obtained, which in turn causes injection molding defects such as short mold and weld, and also causes variations in the shape or the density of the resultant molded bodies. Binder Loadings are, for example, between 10 percent by weight to 100 percent by weight, based on 100 percent by weight of ingredients, or may be 20 percent by weight to 50 percent by weight. The blending ratio of binders to ingredients is 25 to 70 volume percent, preferably 30 to 55 volume percent, and more preferably 35 to 45 volume percent.

### Addition of Glass Component

A preferred embodiment of the present invention is a method for producing a bone filling material comprising "ingredient comprising calcium-based material, and material comprising binder", and glass component. As the glass component, the following materials can be used as appropriate: silica glass which is composed mostly of silicon dioxide; borosilicate glass containing 5 to 20 % by weight of B₂O₃; lead glass containing 5 to 40 % by weight of lead; potassium glass containing 5 to 30 % by weight of potassium; Fluoroaluminosilicate glass including sodium fluoride, aluminum fluoride, and the strontium fluoride; or a mixture of one of these glasses and one kind or a mixture of more than one kind of boric acid, lanthanum oxide, gadolinium oxide, niobium oxide, zirconium oxide, and barium. The sinterability of a sintered body is lowered by consciously adding glass components. As a result, minute cracks and porosities are formed on the surface or inside the sintered body, thereby producing a bon filling material which is suitable for culturing cells. As glass component, the following materials or an appropriate mixture thereof may be used: titanium, titanium alloy, cobalt - chromium alloy, stainless steel, alumina, zirconia. Calcium phosphate-based crystals such as apatite (Ca₁₀ (PO₄)₆ O), or calcium phosphate-based crystals such as CaO-SiO₂-MgO-P₂O₅ based crystallized glass may also be used.

It is preferred that the glass component loadings are adjusted as appropriate based on the physical properties required for the bone filling material, but the loadings are, for example, between 1 percent by weight to 20 percent by weight, based on 100 percent by weight of ingredients, or may be 2 percent by weight to 10 percent by weight. The blending ratio of glass components to ingredients is 1 to 20 volume percent, preferably 2 to 10 volume percent, more preferably 3 to 10 volume percent.

### Addition of Salt or Sugar

A preferred embodiment of the present invention is a method for producing a bone filling material comprising "ingredient comprising calcium-based material, and material comprising binder", and salt or sugar (preferably salt). The sinterability of a sintered body is lowered by consciously adding salt or sugar. As a result, minute cracks and porosities are formed on the surface or inside the sintered body, thereby producing a bone filling material which is suitable for cell culture. And, by immersing the obtained bone filling material in water, salt or sugar can be removed, thereby producing a bon filling material which is suitable for cell culture. Well-known salts or sugars can be used as appropriate. Salts which can be dissolved in water but is not thermally decomposed at temperature that thermally decomposes binders, particularly inorganic salt, is preferred. Specific examples of the salts include sodium chloride, potassium chloride, calcium chloride or calcium carbonate. Well-known sugars such as sucrose, glucose, and fructose can be used as appropriate. Meanwhile, a preferred embodiment of the present invention comprises thermally-degradable components with or without sugar or salt. The word thermally-degradable component represents a component which is not thermally-degraded in the kneading step, but is thermally-degraded in the molding step and the sintering step, or is thermally-degraded at a higher temperature than the heating temperature of the molding step or the sintering step. Since the thermally-degradable components are thermally-degraded somewhere in the molding step, the sintering step, or after the sintering step, a bone filling material having appropriate voids can be obtained.

It is preferred that salt or sugar loadings are adjusted as appropriate based on the physical properties required for the bone filling material, but the loadings are, for example, between 1 percent by weight to 20 percent by weight, based on 100 percent by weight of ingredients, or may be 2 percent by weight to 10 percent by weight. The blending ratio of salt or sugar to ingredients is 1 to 30 volume percent, preferably 2 to 20 volume percent, more preferably 3 to 10 volume percent. When thermally-degradable components are added to ingredients, the same amount as salt or sugar is preferred to be added.

### Kneading

In the kneading step, compound which is a material for injection molding is obtained by mixing the above described ingredient powders and binders. If ingredient powders are not uniformly mixed, several problems will be caused. For example, the geometry of a molded body will be deteriorated. In particular, it is preferred that each bone filling material of the present invention have constant geometry in order to maintain the same dosage of pharmaceutical agents. So it is desirable that the ingredients be made as uniform as possible.

The temperature of the kneading step is preferred to be adjusted as appropriate based on the kind of binders. But if the temperature is low, the ingredient powders and the binders can not be mixed, and if the temperature is high, the binders will be thermally decomposed. So the temperature is set to be, for example, from 110°C to 240°C, preferably from 130°C to 190°C, and more preferably from 140°C to 160°C.

In the kneading step, it takes long time for kneading ingredients uniformly. But if the kneading time is too long, the binders will be thermally decomposed. So the kneading time is preferred to be adjusted as appropriate based on the kinds on binders. The kneading time is, for example, from 30 minutes to 5 hours. It may also be 45 minutes to 1.5 hours.

As a kneading machine used in the kneading step, for example, a pressure type kneader, or a uniaxial or biaxial extrusion kneader can be used as appropriate. Since the bone filling material of the present invention is pharmaceutical agent which will be used for transplant, it is preferred that the bone filling material be free of impurities such as broken pieces of kneading blades of a kneading machine. From this perspective, it is desired that the kneading blades be made of material with high hardness, and kneading blades which are provided by surface protective layers (e.g., deposited by TiN coating) are preferred.

A preferred kneading process is, for example, as follows. Firstly, a kneading machine is heated to a predetermined temperature, and binder having high melting point is cast into the kneading machine. When the binder is dissolved to a certain extent, ingredient powders are cast into the kneading machine. After that, binder having low melting point and ingredient powders are cast into the kneading machine, and 1/2 to 4/5 by volume of ingredient is cast into the kneading machine, followed by casting low-volatile components such as DBP (dibutyl phthalate). And then, the remaining ingredient is cast into the kneading machine. In this way, the aggretation of the ingredient powders could be dispersed by kneading binder having high melting point (high-viscous binder) and ingredient powders in the beginning of the step.

In particular, for example, the kneading step comprises the steps of: putting the (meta) acrylic-based resin and the ethylene-vinyl acetate copolymer in a kneading machine; putting the ingredient, the paraffin wax, and the stearic acid in the kneading machine while kneading the (meta) acrylic-based resin and the ethylene-vinyl acetate copolymer; and putting the dibutyl phthalate in the kneading machine while kneading the (meta) acrylic-based resin, the ethylene-vinyl acetate copolymer, the paraffin wax, and the stearic acid. In this way, the compound which is material for injection molding can be obtained.

However, since the bone filling material of the present invention is replaced with bones in the future, molded products having consciously made minute cracks may be used to promote the replacement. From this perspective, for example, the kneading time may be from 15 minutes to 30 minutes, and the kneading temperature may be from 80°C to 100°C.

### Molding Step

The molding step is a process for producing a molded body with a predetermined shape by injection molding. It is preferred that a bone filling agent have four protruding parts extending from the center of the regular tetrahedron form of the bone filling material toward each vertex thereof. Herein after an example of a mold for producing the bone filling material is explained. Fig. 1 is a conceptual diagram showing an example of a mold used in the method for producing a bone filling material of the present invention. Reference number 1 of Fig. 1 represents the shape of the mold with its grooves get together. Reference number 2 represents a part corresponding to an injection hole. Reference number 3 represents a parting face. And reference number 4 represents a wedge. The above described mold, for example, has: a fixed mold having an inlet (gate) where material is injected; and a movable mold which is contacted with the fixed mold when the material is injected, and is apart from the fixed mold after a molded product is formed. And the inlet of the mold is located at a tip part of one of the protruding parts so that the material for injection molding is injected therefrom, and the parting face of the fixed mold and the movable mold is located at the other three protruding parts, wherein the movable mold has ejector pins located inside the other three protruding parts. The term "inside the other three protruding parts" means the direction from the bottom of the Fig. 1(A) to the reference number 2.

The bone filling material above described has a plurality of protruding parts (e.g., more than four protruding parts). And the mold comprises: a fixed mold having an inlet where material is injected; a movable mold being contacted with the fixed mold when the material is injected, the movable mold being apart from the fixed mold after a molded body is formed, wherein the inlet of the fixed mold is located at a tip part of one of the protruding parts so that the material for injection molding is injected from the tip part of the protruding part, wherein a parting face of the fixed mold and the movable mold has an inclined surface, the inclined surface inclines toward the inlet of the fixed mold from the edge face to the center of the parting face, and wherein the movable mold has dent parts for providing predetermined wedges to the movable mold. As shown in Fig.1, the parting faces provided at the parts corresponding to the protruding parts are not horizontally arranged but are inclined toward the inlet of the mold as it extends to the center of the protruding parts.

Producing a bone filling material with one molding process accompanies difficulties. Those who skilled in the art think of a method for injection molding in which protruding parts independently produced are combined together. But since the bone filling material of the present invention is minute, it is difficult to produce the protruding parts separately and combine them together. The mold of the present invention can produce an injected body having a plurality of protruding parts in one injection molding operation. The mold of the present invention also made it easy for a molded body to be taken out of the fixed mold, for example, by providing a three-dimensional parting face, wherein the parting face of the fixed mold and the movable mold has an inclined surface toward the inlet from the edge to the center of the parting face. For example, when a split mold having a flat parting face is used to mold a molded body, the molded body is highly likely to be stuck in a fixed mold, and it is difficult to remove the molded body from the fixed mold. But the above described mold having inclined parting face lowered the possibility of a molded body remained in a fixed mold.

A preferred embodiment of a mold of the present invention has wedges (4) with the height of 1 µm to 1x10² µm (preferably 5 µm to 2 x 10 µm, or 5 µm to 1 x 10 µm). The shape of the wedge is not specifically limited, if it served as a wedge, and well-known shape of wedge can be adopted. In Fig. 1, three wedges are provided at the parts corresponding to the tip of each protruding part. But the number or the position of the wedges is not particularly limited, and the wedges may be provided at the parts other than the tips of the protruding parts (e.g., the body part of the protruding part). If a molded body remains in the fixed mold, bone filling materials can not be produced. The wedge part serves as a wedge to the moving part, which prevents a molded body being left in the fixed mold when the mold is opened.

A preferred embodiment of the present invention is one of the above described methods for producing a bone filling material, wherein the movable mold has one or a plurality of grooves (preferably parallel grooves or spiral-shaped grooves) with the depth of 1 µm to 2 x 10 µm (preferably 5 µm to 1 x 10 µm). If a molded body remains in the fixed mold, bone filling materials can not be produced. The groove parts increase the surface area of the movable mold part, thereby preventing a molded body from being left in the fixed mold when the mold is opened. The groove part is provided, for example, on the body part of the protruding parts.

A preferred embodiment of the present invention is one of the above described methods for producing a bone filling material, wherein the bone filling material has four protruding parts and the movable mold has ejector pins located inside the protruding parts. It is very important for injection molding to remove a molded body from a mold. In this embodiment, a protruding pin is arranged so that it is located inside a protruding part (so that the pin is directed from the other end of the tip of a protruding part through the center of the mold toward the tip of the protruding part.). So a molded body can be taken out effectively.

A preferred embodiment of the present invention is one of the above described methods for producing a bone filling material, wherein the bone filling material has a plurality of protruding parts. And the mold comprises: a fixed mold; a movable mold being contacted with the fixed mold when material is injected, the movable mold being apart from the fixed mold after a molded body is formed, wherein an inlet of the material for injection molding is located at a parting face of the fixed mold and the movable mold, wherein the parting face of the fixed mold and the movable mold has an inclined surface, the inclined surface inclines toward the center of the bone filling material from the edge face to the center of the parting face, and wherein the movable mold has groove parts for providing predetermined wedges to the movable mold. A molded body having a plurality of protruding parts can be obtained in one injection molding operation by an ingenious mold at the timing of injection molding.

In the molding step, an injection molding is performed preferably by using an injection molding machine. The injection molding machine is not specifically limited, and a well-known injection molding machine can be used. The examples of the injection molding machine include: a vertical injection molding machine or a horizontal injection molding machine; a high pressure injection molding machine, a moderate pressure injection molding machine, or a low pressure injection molding machine; or a plunger injection molding machine or a screw injection molding machine. However, in order to produce a bone filling material having minute protruding parts from calcium phosphate-based material, an injection molding machine which is a horizontal screw type injection molding machine (which is preferably a high pressure injection molding machine) can be preferably used. However, if impurities such as broken pieces of a screw cylinder are mixed in the bone filling material of the present invention, (although it will not be a problem for an ordinary molded body) it will be a problem because the bone filling material is intended to be administered in vivo. So it is preferred that surface protective layers such as TiN coating layers are preferred to be formed on the surface of the screw.

### Binder Removal Step

In the binder removal step, binder contained in the molded body obtained in the above described molded step is removed, and thereby degreased body is produced. The binder removal step is also referred to as a degreasing step. If binder is not removed sufficiently in the binder removal step, the molded body may be cracked or bloated in the sintering step below. In the degreasing step, it is expected that the binder removal is completed without causing defects on the molded body such as deformations and cracks. The examples of binder removal method include the sublimation method, the natural drying method, the solvent extraction method, the thermal degreasing method, and the like, and the thermal degreasing method is preferred. The thermal degreasing method is performed in an ambient atmosphere, a reduced pressure atmosphere, a pressurized atmosphere, a gas atmosphere, or the like, and is preferably performed in an ambient atmosphere. A molded body is preferably placed on a ceramics setter (porous and dense) when it is cast into a degreasing furnace. If the molded body is large (i.e., thick molded body), porous setter such as alumina setter is preferred. It is also desirable to watch for impurities of contaminated setter and components of a heated setter.

A binder removal step, for example, has several stages of heating-up period and duration period in accordance with the pyrolysis temperature of resin contained in binder. In particular, effective pyrolysis of resin having low pyrolysis temperature improves sintering performance. In the present invention, since temperature is raised as above described, resin having low pyrolysis temperature can be effectively pyrolyzed. A preferred embodiment of a bone filling material of the present invention may include compounds having poor biocompatibility in binder, although the bone filling material is administered in vivo. Such compound tends to be a binder having low melting point. So in the heating-up step, in order to vaporize binder having low melting point completely, it is preferred that the temperature be raised relatively moderately. A specific example of heating up ratio is at 1 °C/hour to 3x10²°C/hour until the temperature reaches in the range of 110°C to 300°C which is the temperature of the first maintaining period (preferably until the temperature reaches in the range of 230°C to 250°C), preferably at 1x10 °C/hour to 2x10²°C/hour, more preferably at 2x10 °C/hour to 5x10 °C/hour, and the ratio may also be at 3x10 °C/hour to 4x10 °C/hour. The maintaining step is, for example, from 2x10 minutes to 5 hours, preferably from 3x10 minutes to 2 hours.

### Sintering Step

The sintering step is a step for heating a molded body obtained in the binder removal step. Japanese Patent Laid-Open No. 2004-97259 (paragraph [0025]) shows a sintering at 1,250 °C for an hour. But in a preferred embodiment of the present invention, a molded body is heated from ambient atmosphere to the highest temperature 9x10²°C to 1.1x103°C. This is, for example, to turn α-TCP, as ingredient, effectively into β-TCP. High temperature maintaining period is, for example, 5x10⁻¹ hours to 3 hours. It is noted that in the sintering step, a heating-up step (and maintaining step) is followed by a cooling step, wherein well-known cooling methods are used as appropriate. The sintering period including the cooling period is, for example, from 6 hours to 5x10 hours, preferably from 1x10 hours to 3x10 hours. The molding temperature is, for example, from 1x10²°C to 1.5x10²°C. And the mold temperature is, for example, from 1x10°C to 3x10°C.

### Aftertreatment Step

The aftertreatment step is an optional step for aftertreatment of the molded body obtained in the sintering step. Specific examples of the aftertreatment step include patching holes caused by ejector pins, and cleaning the molded body.

It is another preferred embodiment of the present invention to add well-known pharmaceutical agent in addition to ingredient powder. The bone filling material containing the pharmaceutical agent serves as carriers thereof because the volume of the bone filling material produced in the present invention is approximately uniform. It is preferred that the pharmaceutical agent added to the bone filling material remain activity at high temperatures.

### Impregnation and Administration of Pharmaceutical Agent

Another preferred embodiment of the present invention is a bone filling material (or a sintered body obtained in the sintering step) to which a pharmaceutical agent is impregnated or administered as needed. The methods for administering pharmaceutical agent includes immersion administration, spray administration, and spin coat administration, wherein pharmaceutical composition which is obtained by dissolving pharmaceutical agent with well-known pharmaceutically acceptable diluent (solvent) is administered. Among them, immersion administration is preferred. Immersion administration of pharmaceutical agent impregnates the surface or inside of the bone filling agent with pharmaceutical agent. Namely, the present invention can provide a bone filling material to which a predetermined pharmaceutical agent is impregnated or administered.

A preferred embodiment of the bone filling material of the present invention is the above described bone filling material wherein the pharmaceutical agent comprises an osteogenesis/chondrogenesis promoter (including chondrogenesis promoting factor), a joint disease therapeutic agent, a preventive and/or therapeutic agent for bone/cartilage disease, a bone-regenerating agent, a bone resorption-suppressing substance, an angiogenesis promoter, an antibacterial agent, an antibiotics, or an anticancer agent. A preferred embodiment of the bone filling material of the present invention is the above described bone filling material wherein the pharmaceutical agent comprises thienoindazole derivative represented by the below described general formula (I). The thienoindazole derivative (4,5- dihydro -1 - methyl - 1H- thieno [3,4-g] indazole derivative) can be produced in accordance with a method disclosed in the Japanese Patent Laid-Open No. 2002-356419. It is desirable that effective dose of the pharmaceutical agent that provides a predetermined efficacy is contained in the bone filling material of the present invention. Namely, since well-known pharmaceutical agent can be used in the present invention, the amount of pharmaceutical agent contained in the bone filling material is preferred to be adjusted as appropriate so that the effective dose of the pharmaceutical agent that provides a predetermined efficacy can be administered.

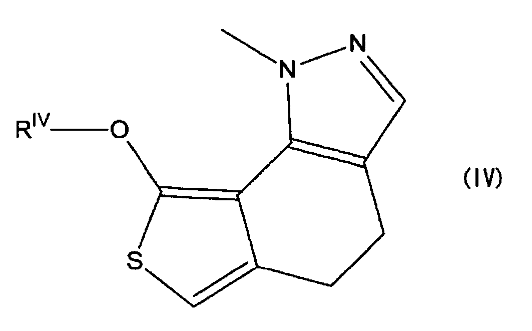

wherein R^{1V} represents a carboxyamido group (-CH (NH₂) (CO₂H), -CH (NH₂) (SO₃H), -CH (NH₂) (SO₂H), -CH (NH₂) (SO₂NHR^{II}), -CH (NH₂) (PO (NH₂) OH) and -CH (NH₂) (PO (OR^{II}) OH), (where R^{II} is a C₁₋₅ linear alkyl group). Among them, carboxyamido group is the most preferred.

### Osteogenesis/Chondrogenesis Promoter

As the osteogenesis/chondrogenesis promoter, a publicly known agent for inducing osteogenesis or chondrogenesis can be used as needed. In particular, a chondrogenesis promoter is, for example, a 2-[1-(2,2- Diethoxy-ethyl) -3-(3-p-tolyl-ureido) -2, 3-dihydro-1H-indol-3-yl] -N-p-tolyl-acetamide which is disclosed in WO 02/087620. As a chondrogenesis promoter, for example, osteogenesis promoting factor can be used. The osteogenesis promoting factor is generally referred to as BMP (bone morphogenetic protein). The BMP is a substance for bone/cartilage induction which acts on undifferentiated mesenchymal cells from outside, differentiating them to chondrocyte or osteoblasts. As the osteogenesis promoting factor, for example, BMP1 to 13 can be used. The BMP used as a pharmaceutical agent of the present invention may be either one of the BMP obtained by genetic recombination or the purified BMP taken form Dunn osteogenic sarcoma (see Takaoka, K., Biomedical Research, 2 (5) 466-471 (1981)).

### Joint Disease Treating Agent

Examples of the joint disease treating agent include: anti-inflammatory steroid agents such as p38MAP kinase inhibitor (thiazole-based compound etc., disclosed in WO00/64894), matrix metalloprotease inhibitor (MMPI), prednisolone, hydrocortisone, methylpredinisolone, dexabethamethasone, and bethamethasone; and non-steroidal anti-inflammatory analgesic agents such as indometacin, diclofenac, loxoprofen, ibuprofen, piroxicam, and sulindac.

### Bone/Cartilage Disease Preventing or Treating Agent

Examples of the bone/cartilage disease preventing or treating agent include one or mixtures of more than one kind of the following substances: non-peptide osteogenesis-promoting substances such as prostaglandin A1 derivative, vitamin D derivative, vitamin K₂ derivative, eicosapentaenoic acid derivative, benzylphosphonic acid, bisphosphonic acid derivative, sex hormone derivative, phenolsulfophthalein derivative, benzothiopyran or benzothiepine derivative, thienoindazole derivative, menatetrenone derivative, helioxanthine derivative; and a hardly soluble peptide osteogenesis-promoting substance. These substances can be obtained by a known method. A bone/cartilage disease preventing agent includes one or both of a cartilage disease preventive agent and an agent preventing the situation that a bone/cartilage disease develops.

### Bone-Regenerating Agent

Examples of the bone-regenerating agent include one kind or a mixture of more than one kind of the following substances: calmodulin; actinomycin D; cyclosporin A; glucosamine sulfate; glucosamine hydrochloride; marrow extract; calcium phosphate; lactic acid/ glycolic acid/ ε-caprolactone copolymer; platelet rich plasma; or human marrow mesenchymal cell. These substances can be obtained by a known method.

### Bone Resorption-Suppressing Substance

Examples of the bone resorption-suppressing substance include one kind or a mixture of more than one kind of estrogenic agent, calcitonin, and bisphosphonate. These substances can be obtained by a known method.

### Angiogenesis Promoter

Examples of the angiogenesis promoter include one kind or a mixture of more than one kind of the following substances: indigocarmine; 4- [N-methyl-N-(2-phenylethyl) amino] -1- (3, 5-dimethyl-4-propionyl aminobenzoyl) piperidine; 4-(5H-7, 8, 9, 10- tetrahydro-5, 7, 7, 10, 10-pentamethyl benzo [e] naphtho [2, 3-b] [1,4] diazepine-13-yl) benzoic acid; activated protein C; urotensin II -like peptide compound; fibroblast growth factor (FGF) (including basic FGF and acidic FGF); vascular endothelial cell growth factors (VEGF) (preferably a platelet-derived factor); hepatocyte growth factor (HGF); angiopoetin (including angiopoetin-1 and angiopoetin-2); platelet-derived growth factor (PDGF), Insulin-like growth factor (IGF) or smooth muscle embryo myosin heavy chain(SMemb). Of these substances, fibroblast growth factor is preferred (see, Hockel, M. et al., Arch. Surg., No. 128, p. 423, 1993). A basic fibroblast growth factor (bFGF) is preferred as a fibroblast growth factor, and a specific example includes trafermin (gene recombinant). Namely, a preferred embodiment of the bone filling material of the present invention is the above described bone filling material comprising trafermin, a salt thereof, a solvate thereof, or a prodrug thereof. "A salt thereof" represents a salt of trafermin, and is specifically the same salt as above explained. "A solvate thereof" represents a solvate of trafermin, and is specifically the same solvate as above explained. "A prodrug thereof" represents a prodrug of trafermin, and represents an agent that turns into, for example, trafermin, an ion thereof, or a salt thereof in vivo after administration. In particular, a prodrug contains protecting groups such as an amino group which are taken off in vivo, and acts the same as trafermin.

### Antibacterial Agent or Antibiotics

As an antibacterial agent or an antibiotic, a well-known antibacterial agent or antibiotic can be used as appropriate. Specific examples of antibacterial agents or antibiotics include one kind or a mixture of more than one kind as appropriate of the following substances: sulfonamide such as sulfacetamide, sufamethizol, sulfadimidine, and sulfamerazine; chloramphenicols antibacterial agent such as chloramphenicol (CP), and tiamphenicol; quinolones antibacterial agent such as ofloxacin (OFLX), ciprofloxacin (CPFX), enrofloxacin, lomefloxacin (LFLX), rufloxacin, levofloxacin (LVFX), fleroxacin (FLRX), nadifloxacin (NDFX), norfloxacin (NFLX), and sparfloxacin (SPFX); fusidic acid (FA); fusafungine; fosfomycin (FOM), mupirocin (MUP); brodimoprim; dirithromycin; penicillins antibacterial agent such as benzylpenicillin (PCG); penicillin G procaine; benzathine penicillin, phenoxymethylpenicillin (Penicillin V), methicillin, ampicillin (ABPC), cloxacillin (MCIPC), carbenicillin, pivampicillin (PVPC), amoxicillin (AMPC), talampicillin (TAPC), bacampicillin (BAPC), ticarcillin (TIPC), azlocillin, mezlocillin, pivmecillinam (PMPC), piperacillin (PIPC), amoxicillin (AMPC) - clavulanic-acid (CVA) (co-amoxiclav), apalcillin, temocillin, ticarcillin-clavulanic acid (CVA), ampicillin (ABPC) - sulbactam (SBT), sultamicillin (SBTPC), and piperacillin (PIPC) - tazobactam (TAZ); streptomycins antibiotics such as streptomycin (SM); tetracyclines antibiotics such as chlortetracycline, aureomycin, chloramphenicol (CP), oxytetracycline (OTC), demethylchlortetracycline, demeclocycline, ledermycin®, lymecycline, doxycycline (DOXY), and minocycline (MINO); aminoglycosides antibiotic such as neomycin, spectinomycin (SPCM), gentamycin (GM), tobramycin (TOB), amikacin (AMK), micronomicin (MCR), isepamicin (ISP), and arbekacin (ABK); macrolides antibiotics such as erythromycin (EM), spiramycin (SPM), roxithromycin (RXM), azithromycin (AZM), midecamycin (MDM), and clarithromycin (CAM); glycopeptides antibiotics such as vancomycin (VCM), and teicoplanin (TEIC); polypeptides antibiotics such as colistin (CL); streptogramins antibiotics such as virginiamycin, and pristinamycin; lincomycins antibiotics such as clindamycin (CLDM); cephalosporins antibiotics such as cephalexin (CEX), cefazolin (CEZ), cefradine (CED), cefadroxil (CDX), cefamandole (CMD), cefuroxime (CXM), cefaclor(CCL), cefotaxime (CTX), cefsulodin (CFS), cefperazone, cefotiam (CTM), ceftriaxone (CTRX), cefinenoxime (CMX), ceftazidime (CAZ), ceftiroxime, cefonicid, cefpiramide (CPM), cefoperazone (CPZ) - sulbactam (SBT), cefpodoxime (CPDX), cefozidime, cefdinir (CFDN), cefetamet (CEMT), cefpirome (CPR), cefprozil, ceftibufen, and cefepime (CFPM); cephamycins antibiotics such as cefoxitin (CFX), cefmetazole (CMZ), and cefotetan (CTT); oxacephems antibiotics such as latamoxef (LMOX), and flomoxef (FMOX); carbapenems antibiotics such as imipenem (IPM) - cilastatin (CS) (tienam®); monobactams antibiotics such as aztreonam (AZT); carbacephems antibiotics such as loracarbef (LCBF); carbapenems antibiotics such as panipenem (PAPM) - betamipron (BP); ketolides antibiotic such as telithromycin (TEL).

### Anticancer Agent

Anticancer agent is a pharmaceutical agent for treating or preventing cancer. A publicly known anticancer agent can be used as needed. Specific examples of anticancer agent include the following substances: anticancer hemolytic streptococcus formulation such as OK-432 (commercial name Picibanil); anticancer polysaccharide such as krestin, lentinan, schizophyllan, and sonifilan; anticancer antibiotics such as mitomycin C (commercial name Mitomycin, etc.), actinomycin D (commercial name Cosmegen), bleomycin hydrochloride (commercial name Bleo), bleomycin sulfate (commercial name Bleo S), daunorubicin hydrochloride (commercial name Daunomycin), doxorubicin hydrochloride (commercial name Adriacin), neocarzinostatin (commercial name Neocarzinostatin), aclarubicin hydrochloride (commercial name Aclacinon), or epirubicin hydrochloride (commercial name Farmorubicin); mitotic inhibitor such as Vinblastine; alkylating agent such as cis-platin, carboplatin, and cyclophosphamide; antimetabolite such as 5-fluorouracil, cytosine arabinoside and hydroxyurea, N- {5- [N-(3, 4-dihydro-2-methyl-4-oxoquinazoline-6-ylmethyl) -N-methylamino] -2- thenoyl} -L-glutamic acid; anticancer antibiotics such as adriamycin and bleomycin; enzyme such as asparaginase; topoisomerase inhibitor such as etoposide; biological response modifier such as Interferon; antiestrogen such as "NOLVADEX" (tamoxifen); antiandrogen such as "CASODEX"; antimetabolite such as Fluorouracil, Tegafur, Tegafur-uracil, and Methotrexate; plant alkaloid such as Vncristine; anticancer antibiotic such as mitomycin C, actinomycin D, bleomycin hydrochloride, bleomycin sulfate, daunorubicin hydrochloride, doxorubicin hydrochloride, neocarzinostatin, aclarubicin hydrochloride, Aclacinon, and epirubicin hydrochloride; and platinum complex such as cyclotriphosphazene-platinum complex, and cisplatin-platinum complex.

It is expected that the bone filling material of the present invention promotes its replacement with bone tissues in vivo, so pharmaceutical agents including a specific polypeptide or gene may be administered or impregnated to the bone filling material. The examples of the polypeptide or the gene include a basic fibroblast growth factor (bFGF), a platelet derived growth factor (PDGF), insulin, an insulin-like growth factor (IGF), a hepatocyte growth factor (HGF), a glial cell line-derived neurotrophic factor (GDNF), a neurotrophic factor (NF), hormone, cytokine, a bone morphogenetic factor (BMP), a transforming growth factor (TGF), a vascular endothelial cell growth factor (VEGF). Among them, a growth factor promoting neoangiogenesis and/or osteogenesis is preferred. The examples of the growth factor include a bone morphogenetic factor (BMP), a bone growth factor (BGF), a vascular endothelial cell growth factor (VEGF) and a transforming growth factor (TGF). The specific example is a calponin gene disclosed in the Japanese Patent No. 3713290. The gene is preferred to be contained in the bone filling material as much as the amount which is effective for the gene therapy. It is also preferred that the gene be contained in the bone filling material as it is (naked), in a micelle state, or in the form of a recombinant vector which is transformed into a known vector such as a virus vector. The pharmaceutical agent may be a known genetic antibody.

The gene can be adjusted based on well-known base sequence in accordance with ordinary methods. For example, cDNA of the targeted gene is adjusted in the following method. RNA is extracted from osteoblasts, and primer is produced based on a well-known base sequence, and then cloning by PCR method. Also commercially available genes may be used.

A preferred embodiment of a bone filling material of the present invention is the above described bone filling material including stabilizer. As the stabilizer, a well-known stabilizer used for polymer compound, in particular, pharmaceutically acceptable stabilizing agents can be used as appropriate. The strength of the bone filling material of the present invention is maintained mainly in vivo for extended period. But it is assumed that the bone filling material is decomposed in the early stages due to the existence of enzymes such as protease. So a preferred embodiment of the present invention includes inhibitors such as protease inhibitor. Well-known enzyme inhibitor can be used for the inhibitor as appropriate. The specific examples include one or more than one kind of the following substances: 4-(2- aminoethyl) benzene sulfonyl fluoride, aprotinin, bestain, calpains inhibitor I, calpains inhibitor II, chymostain, 3,4-Dichloroisocoumain, E-64, EDTA, EGTA, lactacystin, leupeptin, MG-115, MG-132, pepstain A, phenylmethyl sulfonyl fluoride, proteasome inhibitor I, p- toluene sulfonyl - L - lysine chloromethylketone, p- toluene sulfonyl - L - phenylalanine chloromethylketone, or tyrosine inhibitor. These protease inhibitors are commercially available, and the inhabitory concentrations thereof are also commonly known. A preferred embodiment of the compound formed by the bone filling material of the present invention maintains the strength for a prolonged period and has sustained drug release. Therefore, the bone filling material of the present invention preferably contains 2 to 100 times of one dosage of the above protease inhibitor, more preferably contains 2 to 50 times thereof. The specific dose level of the protease inhibitor differs based on the kind of the protease inhibitor to be used. The dose preferably contains the amount of protease inhibitor that makes inhibitor's function effective (i.e., the effective dose). In general, the bone filling material (per 1 g) contains 0.1 µg to 0.5 mg of protease inhibitor. The amount included may be 1 µg to 0.1 µg, or may be 10 µg to 0.1 µg. The specific amount of dosage increases in almost proportion to the volume of the site to which the bone filling material is administered.

### Adhesiveness-imparting Agent

Another preferred embodiment of the present invention is a bone filling material (or, a sintered body obtained in the sintering process) to which adhesiveness-imparting agents are impregnated or administered as appropriate. When a heat-resistant adhesiveness-imparting agent is used, the adhesiveness-imparting agent may be mixed with ingredient powders so that a bone filling material which is powder blended with the adhesiveness-imparting agent can be obtained (in this case, the adhesiveness-imparting agent exists on the surface of the bone filling material, and when the adhesiveness-imparting agent on the surface is replaced with bone tissues, a new surface having the other adhesiveness-imparting agent appears, thereby maintaining the adhesiveness of the bone filling material). Also, a powdered adhesiveness-imparting agent may be sprayed on the surface of a molded body or a sintered body. Furthermore, adhesiveness-imparting agents may be added to the surface of the bone filling material by powder blending, wherein a plurality of bone filling agents and powdered adhesiveness-imparting agents are mixed together and then agitated as needed. The adhesiveness-imparting agent may be impregnated or administered with the above mentioned pharmaceutical agent, and the adhesiveness-imparting agent alone may be impregnated or administered. The adhesiveness-imparting agent is an agent for raising adhesion property between the bone filling materials, and it is preferred that the adhesiveness-imparting agent alone do not have high adhesiveness but increase adhesiveness when it contacts with cells in vivo. A specific example of the adhesiveness-imparting agent is Thrombin. Thrombin is one of enzymes which promotes blood clot. Thrombin produces fibrin which is a blood clotting substance in vivo. Fibrin produced by thrombin promotes blood clotting. So when thrombin is used as an adhesiveness-imparting agent, the adhesiveness of the bone filling material will be improved, which raises the strength of the bone filling material by fixing the bone filling materials each other firmly. Thrombin can be impregnated or administered with the same amount of the above described pharmaceutical agent and in the same manner thereof.

A preferred embodiment of the present invention comprises the step of: preparing a composition including two kinds of adhesiveness-imparting agents; obtaining a first bone filling material group by impregnating a certain bone filling material group with a first composition or by administering a first composition to a certain bone filling material group; obtaining a second bone filling material group by impregnating a certain bone filling material group with a second composition or by administering a second composition to a certain bone filling material group, wherein the bone filling material groups including the first bone filling material group and the second bone filling material group are used as the bone filling materials. And for example, the first composition comprises: one or more than one kind of compound represented by the below-described general formula (I) or (II), or a first compound including 3 to 8 repeating units represented by the below-described general formula (III); and a first diluent (or a carrier), and the second composition comprises: one or more than one kind of compound represented by the below-described general formula (I) or (II), or a second compound including 3 to 8 repeating units represented by the below-described general formula (III); and a second diluent.

X₁-(OCH₂CH₂)ₙ-X₂ (I)

wherein X₁ and X₂ are the same or different and each represents -R¹COONHS (where R¹ is a C₁₋₇ alkylene group), -COR¹COONHS(where R¹ is a C₁₋₇ alkylene group), -NOCOR¹-R² (where R¹ is a C₁₋₇ alkylene group, and R² is a maleimide group), -R¹NH₂ (where R¹ is a C₁₋₇ alkylene group), -R¹SH (where R¹ is a C₁₋₇ alkylene group), or -CO₂PhNO₂ (where Ph is an o-, m-, or p-phenylene group), and n is an integer of 80 to 1000.

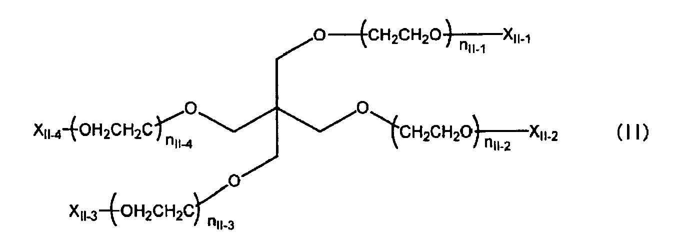

wherein X_{II-1} to X_{II-4} are the same or different and each represents -R¹COONHS (where R¹ is a C₁₋₇ alkylene group), -COR¹COONHS (where R¹ is a C₁₋₇ alkylene group), -NOCOR¹-R² (where R¹ is a C₁₋₇ alkylene group, and R² is a maleimide group), -R¹NH₂ (where R¹ is a C₁₋₇ alkylene group), -R¹SH (where R¹ is a C₁₋₇ alkylene group), or -CO₂PhNO₂ (where Ph is an o-, m-, or p-phenylene group), and n_{II-1} to n_{II-4} are the same or different and each represents an integer of 20 to 250.

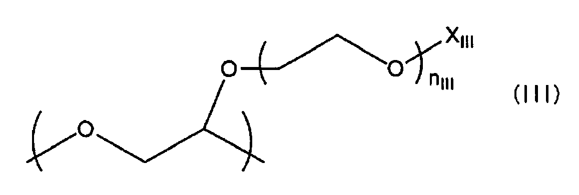

wherein X_{III} represents -R¹COONHS (where R¹ is a C₁₋₇ alkylene group), -COR¹COONHS, -NOCOR¹-R² (where R¹ is a C₁₋₇ alkylene group, and R² is a maleimide group), -R¹NH₂ (where R¹ is a C₁₋₇ alkylene group), -R¹SH (where R¹ is a C₁₋₇ alkylene group), or -CO₂PhNO₂ (where Ph is an o-, m-, or p-phenylene group), and n_{III} represents an integer of 10 to 150.

In the general formula (I), X₁ and X₂ are the same or different and each represents -R¹COONHS, -COR¹COONHS, -NOCOR¹-R² (where R² is a maleimide group), -R¹NH₂, -R¹SH, or -CO₂PhNO₂, and n is an integer of 80 to 1000. In the general formula (I), X₁ and X₂ preferably are the same. R¹ is a C₁₋₇ alkylene group, preferably is a C₁₋₅ alkylene group, and more preferably is a C₁₋₂ alkylene group or a C₅ alkylene group. Ph is an o-, m-, or p-phenylene group, and preferably is a p-phenylene group. And, n is an integer of 80 to 1000, and preferably is 100 to 500.

In the general formula (II), X_{II-1} to X_{II-4} are the same or different and each represents -R¹COONHS, -COR¹COONHS, -NOCOR¹-R² (where R² is a maleimide group), -R¹NH₂, -R¹SH, or -CO₂PhNO₂, and n_{II-1} to n_{II-4} are the same or different and each represents an integer of 20 to 250. In the general formula (II), X_{II-1} to X_{II-4} preferably are the same. R¹ is a C₁₋₇ alkylene group, preferably is a C₁₋₅ alkylene group, and more preferably is a C₁₋₂ alkylene group or a C₅ alkylene group. Ph is an o-, m-, or p-phenylene group, and preferably is a p-phenylene group. And n_{II-1} to n_{II-4} preferably are the same and each represents an integer of 20 to 250, and preferably is an integer of 40 to 200.

In the general formula (III), X_{III} represents -R¹COONHS, -COR¹COONHS, -NOCOR¹-R² (where R² is a maleimide group), -R¹NH₂, -R¹SH, or -CO₂PhNO₂, and n_{III} represents an integer of 10 to 150. In the general formula (III), R¹ is a C₁₋₇ alkylene group, preferably is a C₁₋₅ alkylene group, and more preferably is a C₁₋₂ alkylene group or a C₅ alkylene group. Ph is an o-, m-, or p-phenylene group, and preferably is a p-phenylene group. The number of repeating units of the compound represented by the general formula (III) preferably is 3 to 5, more preferably is 4. And the ends of the compound represented by the general formula (III) preferably have functional groups represented by X_{III} of the general formula (III). In particular, it is preferred that the both ends of the compound have a group represented by a formula X_{III}-(OCH₂CH₂) n_{III}-O-CH₂-CH (-O-(CH₂CH₂O)_{nIII}-X_{III})-CH₂- and a group represented by a formula X_{III}-(OCH₂CH₂)_{nIII}-O-CH₂-CH(-O-(CH₂CH₂O)n_{III}-X_{III})-CH₂-O-.

A preferred embodiment of the adhesiveness-imparting agent of the present invention is the above described adhesiveness-imparting agent, wherein the first compound comprises one or more than one kind of compounds represented by the general formula (I) or (II), where X₁, X₂ or X_{II-1} to X_{II-4} are the same or different and each represents -NOCOR¹-R² or -R¹NH₂, and wherein the second compound comprises one or more than one kind of compounds represented by the general formula (I) or (II), where X₁, X₂ or X_{II-1} to X_{II-4} are the same or different and each represents -COR¹COONHS, -R¹SH, or -CO₂PhNO₂ (where Ph is an o-, m-, or p-phenylene group).

The first compound mixed in the first composition preferably has a functional group of -NOCOR¹-R² or -R¹NH₂ (in particular, a functional group of -R¹NH₂). It is also preferred that the second compound, mixed with the first compound, undergoes rapid crosslinkage reaction and the resultant compound has predetermined strength (resilience) and morphological stability. It is also desirable that the compound, which is obtained by mixing the first compound with the second compound, has a certain steric structure and so keep releasing proper amount of a pharmaceutical agent contained therein. From this perspective, it is preferred that the second compound mixed in the second composition have a functional group of -COR¹COONHS, -R¹SH, or - CO₂PhNO₂ (where Ph is an o-, m-, or p-phenylene group) (in particular, a compound having a functional group of -COR¹COONHS). Also, both the first and the second compounds preferably are represented by the general formula (II). In particular, a combination of a propylamine group and a succinimidyl group is preferred as a combination of the functional group of the first compound and that of the second compound.

Another preferred embodiment of the adhesiveness-imparting agent of the present invention comprises the first compound and the second compound, wherein the first compound comprises one or more than one kind of compound represented by the general formula (I) or (II), where X₁, X₂ or X_{II-1} to X_{II-4} are the same or different and each represents -NOCOR¹-R² (where R¹ is a C₁₋₅ alkylene group) or -R¹NH₂, (where R¹ is a C₁₋₅ alkylene group), and wherein the second compound comprises one or more than one kind of compound represented by the general formula (I) or (II), where X₁, X₂ or X_{II-1} to X_{II-4} are the same or different and each represents -COR¹COONHS, -R¹SH (where R¹ is a C₁₋₅ alkylene group), or -CO₂PhNO₂ (where Ph is a p-phenylene group).

The molecular weight of a compound composing the compound is not specifically limited, but a preferred compound can be obtained from a compound of molecular weight (number average molecular weight) 3 x 10³ to 4 x 10⁴, and a more preferred compound can be obtained from a compound of molecular weight 1 x 10⁴ to 3 x 10⁴.

The concentration of a compound is not specifically limited if the concentration of the compound composing the compound in the first composition or the second composition are in the range of 5 mM to 20 mM. A particularly preferred gel can be obtained from the gelator with the above concentration. In particular, it is assumed that a particularly effective compound can be obtained when the concentration of the component in each composition is from 6 mM to 30 mM.

The compound represented by the general formula (I) or (II), and the compound including 3 to 8 repeating units represented by the general formula (III) are commercially available and can be synthesized by a well known method.

The compound represented by the general formula (I) or (II), and the compound including 3 to 8 repeating units represented by the general formula (III) may respectively be a salt thereof or a solvate thereof.

The term "a salt thereof" represents a salt of the above described compounds, particularly represents pharmaceutically acceptable salts of the above described compounds. The term "pharmaceutically acceptable" in this specification means that something is not deleterious to the recipient thereof. The polyphosphoric acid of the present invention can be made to salt by in an ordinary method. The examples of the salt includes: the alkaline metal salts such as sodium salt, potassium salt, and lithium salt; the alkaline earth metal salts such as calcium salt, and magnesium salt; the metal salts such as aluminum salt, iron salt, zinc salt, copper salt, nickel salt, and cobalt salt; the inorganic salts such as ammonium salt; and the organic amine salts such as t-octyl amine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N-dibenzylethylenediamine salt, chloroprocaine salt, procaine salt, diethanolamine salt, N-benzyl-N-phenethylamine salt, piperazine salt, tetramethylammonium salt, tris (hydroxymethyl) aminomethane salt. Among these salts, as polyphosphoric acid salt, alkaline metal salt is preferred, and sodium salt is more preferred. In this specification, "a salt thereof" may include not only anhydrous salt but also hydrate salt. These salts, for example, are ionized in vivo, and act the same as the above described compounds.

The term "a solvate thereof" represents a solvate of the above described compounds. The solvate herein includes a hydrate. The agent of the present invention may absorb moisture, be attached with absorption water, and be hydrated when it is left in the atmosphere or recrystallized. The solvates thus obtained are also included in "a solvate thereof". These solvates are ionized in vivo, and act the same as the above described compounds.

A preferred embodiment of the adhesiveness-imparting agent of the present invention comprises a well known pharmaceutically acceptable diluent as a diluent. Specific examples of the diluent are solvents including one kind or a mixture of more than one kind of water, phosphate buffer solution, citrate buffer solution, phosphate buffered saline or physiologic saline solution.

The acid level of the diluent (or solvent) is not specifically limited, but it includes pH 3 to pH 11. In order to improve the strength of the gel obtained to some extent, the acid level is preferably from pH 5 to pH 10, more preferably form pH 6 to pH 9, and further preferably from pH 7 to pH 8. The molar concentration (M) of the diluent is not specifically limited, but it includes 1 mM to 1 M. In order to improve the strength of the gel obtained to some extent, the molar concentration is preferably from 5 mM to 300 mM, more preferably form 10mM to 200mM, and further preferably from 15 mM to 100 mM.

A preferred embodiment of the adhesiveness-imparting agent of the present invention is the above described adhesiveness-imparting agent wherein one or both of the first composition and the second composition include a stabilizing agent. As the stabilizing agent, a publicly known stabilizing agent used for a polymer etc., in particular, a pharmaceutically acceptable stabilizing agent can be used as needed. The compound obtained from the adhesiveness-imparting agent of the present invention is expected to maintain its strength for a prolonged period mainly in vivo. Since enzymes such as protease exists in vivo, the compound may be dissolved by the enzymes. So a preferred embodiment of the bone filling material of the present invention includes inhibitors such as protease inhibitor. As the inhibitors, publicly known enzyme inhibitors can be used as needed. Specific examples of the protease inhibitor include one or more than one kind of 4-(2-aminoethyl) benzene sulfonyl fluoride, aprotinin, bestain, calpains inhibitor I, calpains inhibitor II, chymostain, 3,4-dichloroisocoumain, E-64, EDTA, EGTA, Lactacystin, Leupeptin, MG-115, MG-132, pepstain A, phenylmethyl sulfonyl fluoride, proteasome inhibitor I, p-toluene sulfonyl-L-lysine chloromethylketone, p-toluene sulfonyl-L-phenylalanine chloromethylketone, or tyrosine inhibitor. These protease inhibitors are commercially available, and the inhibitory concentrations thereof are also publicly known. A preferred embodiment of a compound formed by the adhesiveness-imparting agent of the present invention maintains the strength for a prolonged period and has sustained drug release. So the adhesiveness-imparting agent of the present invention preferably contains 2 to 100 times of one dosage of the above protease inhibitor, more preferably contains 2 to 50 times thereof. The specific dose level of the protease inhibitor differs based on the kind of the protease inhibitor to be used. The dose preferably contains the amount of protease inhibitor that makes inhibitor's function effective (i.e., the effective dose). In general, the adhesiveness-imparting agent (1 mL) contains 0.1 µg to 0.5 mg of protease inhibitor. The amount included may be 1 µg to 0.1 mg, or may be 10 µg to 0.1 mg.

When the above described adhesiveness-imparting agent and a pharmaceutical agent is used to form a compound, the resultant compound comprising the adhesiveness-imparting agent contains the pharmaceutical agent, thereby also acting as the bone filling material having sustained drug release. The degradability of the compound can be controlled by adjusting the structure of the adhesiveness-imparting agent and the amount of stabilizing agent added, which makes it possible to adjust the sustained release of the pharmaceutical agent. Namely, the present invention can also provide a method for controlling release property of a pharmaceutical agent by adjusting the amount of the stabilizing agent added to the adhesiveness-imparting agent.

A preferred embodiment of the present invention is provided with a covering layer on the surface of the bone filling agent. The thickness of the covering layer is preferred to be adjusted as needed, but it is for example 1 µm to 5 x 10 µm. And the covering layer includes biocompatible compounds having hydrophilic groups. Examples of hydrophilic group include an OH group, a COOH group, an NH₃ group, a CO₃ group, and a SO₃ group. It is preferred that the covering layer include compounds having an OH group, which is the same hydrophilic group as that of an apatite (Ca₁₀ (PO₄)₆ O) contained in bones because such a covering layer has affinity with bones, thereby promoting osteogenesis. A silica gel layer can be formed on the surface of the bone filling material in the following way. Firstly, acid aqueous solutions such as hydrochloric acid, nitric acid, and sulfuric acid are mixed in liquid glass (Na₂O SiO₂ nH₂O). And when the mixture showed a proper viscosity, the bone filling material is impregnated with the mixture, and then it is taken out from the mixture and is soaked in water so that Na⁺ ions are dissolved in water. The silica gel layer may also be formed in the following way. Firstly, hydrolysis or polymerization reaction of alkoxide of silicon (or titanium) is promoted by mixing alcohol solution of tetramethoxysilane (titanate), tetraethoxysilane (titanate), tetraproxysilane (titanate), tetraisopropoxysilane (titanate), or tetrabutoxysilane (titanate), which are alkoxide of the silicon (or titanium), with catalysers (acid aqueous solution such as hydrochloric acid, nitric acid, sulfuric acid, and acetic acid, or ammonia water solution) as needed. When the mixture showed a proper viscosity, the bone filling material is impregnated with the mixture, and then it is taken out from the mixture.

### Bone Filling Material

The bone filling material obtained by the above method has high dimensional accuracy and exhibits less unevenness. Also, there are less defective materials produced, and mass production of the bone filling material is made possible by the above method. A preferred embodiment of the bone filling material of the present invention preferably has a shape having a plurality of protruding parts. And it is more preferred that the protruding parts are provided so that they are arranged in linear symmetry, plane symmetry, and spatial symmetry. A specific preferred shape of the bone filling material is a tetrapod-shape (a shape having four protruding parts extending toward each vertex from the center of the regular tetrahedron), or a shape having n (n = 6, 8, 12, etc.) protruding parts extending toward each vertex from the center of the regular body having n faces. The size of the bone filling material (the diameter of a sphere that the bone filling material can be accommodated) is, for example, from 1 x 10⁻² mm to 5 mm, preferably from 5 x 10⁻² mm to 3 mm, further preferably from 1 x 10⁻² mm to 2 mm, more preferably from 2 x 10⁻¹ mm to 1.5 mm. Furthermore, a certain disease can be effectively treated and a proper dosage of pharmaceutical agent can be administered by using bone filling material having predetermined pharmaceutical agent administered on its surface. Hereinafter, preferred shapes of the bone filling material of the present invention are explained.

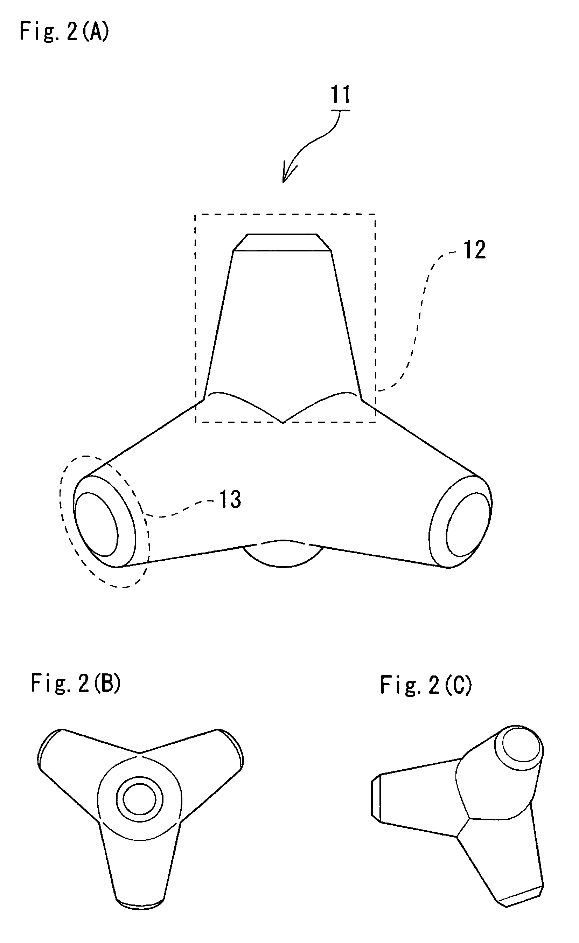

Fig. 2 is a conceptual diagram for explaining an example of a tetrapod-shaped bone filling material. Fig. 2(A) is a side view, Fig. 2(B) is a top view, and Fig. 2(C) is a perspective view. Note that "Fig." means "Figure" (the same below). The bone filling material (11) shown in Fig.2 is a tetrapod-shaped (a shape having four protruding parts (12) extending toward each vertex from the center of the regular tetrahedron) bone filling material. It is preferred that a taper is provided at the tip part (13) of each protruding part (12) and is smoothly shaped (the same below). As shown in Fig.2, each protruding part (12) has substantially the same shape, but one or two of them may be small shaped. Also, each protruding part has, for example, a truncated cone shape which is tapered toward the tip end thereof. The tip portion of each protruding part may be hemispheric (the same below).

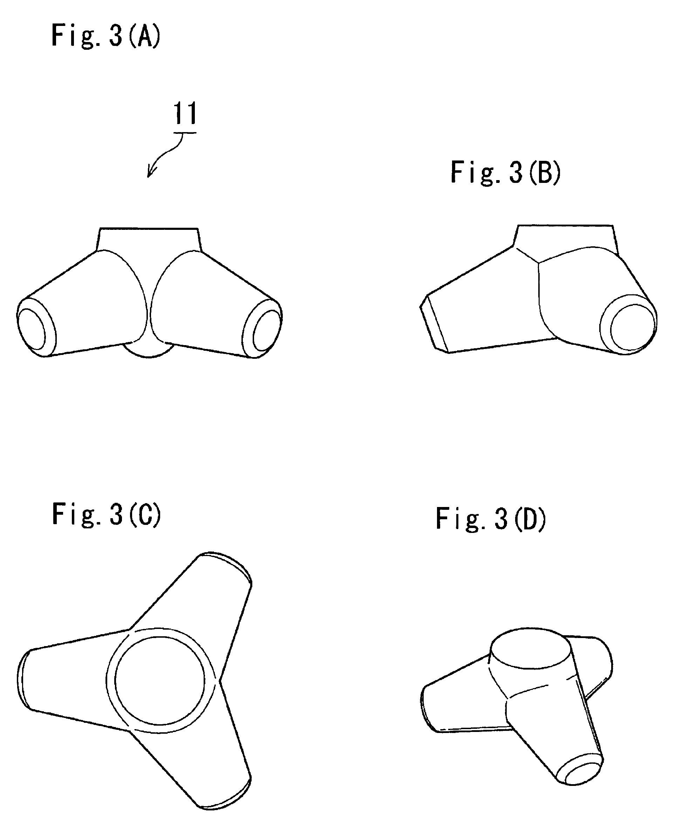

Fig. 3 is a conceptual diagram for explaining an example of a tetrapod-shaped bone filling material with its top portion cut off. Fig. 3(A) and Fig. 3(B) are side views, Fig. 3(C) is a top view, and Fig. 3(D) is a perspective view. The bone filling material according to this embodiment relates to a tetrapod-shaped bone filling material shown in Fig.2 having one protruding part which is cut off in the middle. Specifically, it is a bone filling material having three protruding parts extending toward each vertex from the center of the regular tetrahedron, and a protruding part extending toward the remaining one vertex which is shorter than the other protruding parts. When a plurality of the above shaped bone filling material is used, continuous holes of a cluster of the bone filling material can be made smaller than those of the tetrapod-shaped bone filling material. So the strength of the whole bone filling material can be improved.

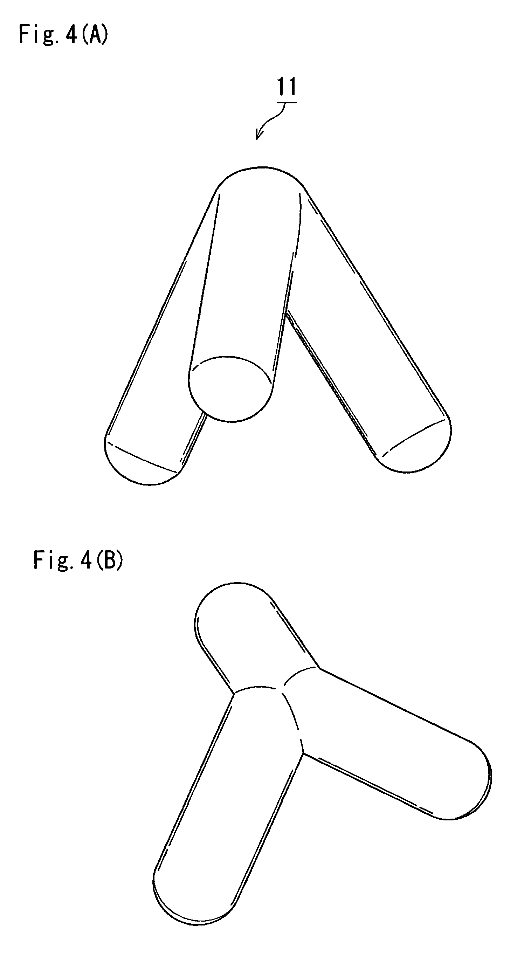

Fig. 4 is a conceptual diagram for explaining an example of a bone filling material having three protruding parts. Fig. 4(A) is a perspective view, and Fig. 4(B) is a top view. It has protruding parts, for example, along the ridge lines of the tetrahedron. And the tip part of each protruding part is, for example, hemispheric. Since the bone filling material according to this embodiment is entangled with each other, strongly bonded bone filling material can be formed.

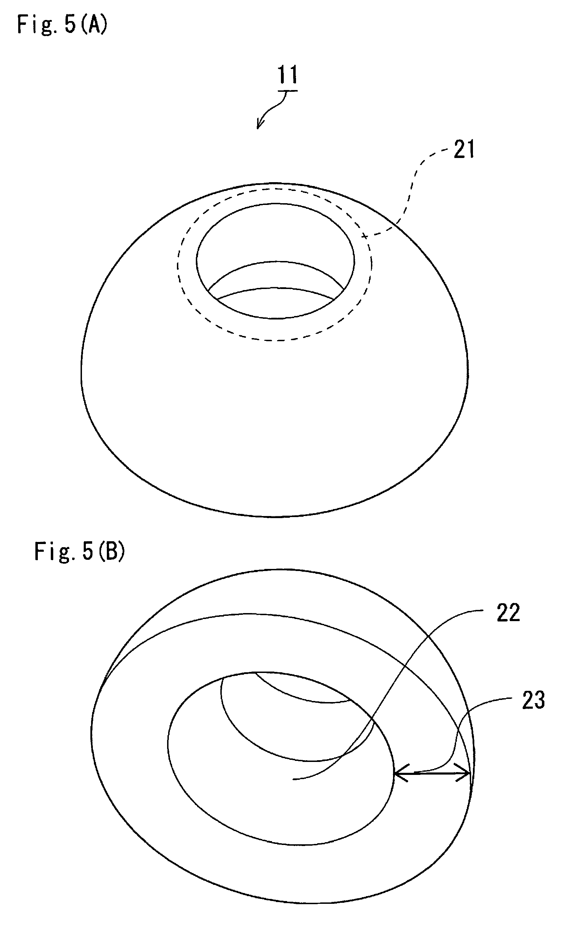

Fig. 5 is a conceptual diagram for explaining an example of a hemispherical bone filling material. Fig. 5(A) is a perspective view, and Fig. 5(B) is a bottom view. The bone filling material according to this embodiment, as shown in Fig. 5(A), is preferred to have a hole part (21) penetrating form the top part to the bottom of the hemisphere. When the bone filling material get together, the hole parts form continuous holes wherein cells can penetrate easily. The shape of the hole part is not limited, but as shown in Fig. 5(A), column shape is preferred. When the radius of the hemisphere is represented by r, the radius of the column-shaped hole is, for example, from r/10 to 2r/3, and is preferably from r/5 to r/2. These preferred holes can maintain the strength of the bone filling material. It is preferred that the bone filling material according to this embodiment have a mortised part (22) in the undersurface part. The example of the thickness (23) of the bone filling material is from r/20 to r/3, and is preferably from r/10 to r/4.

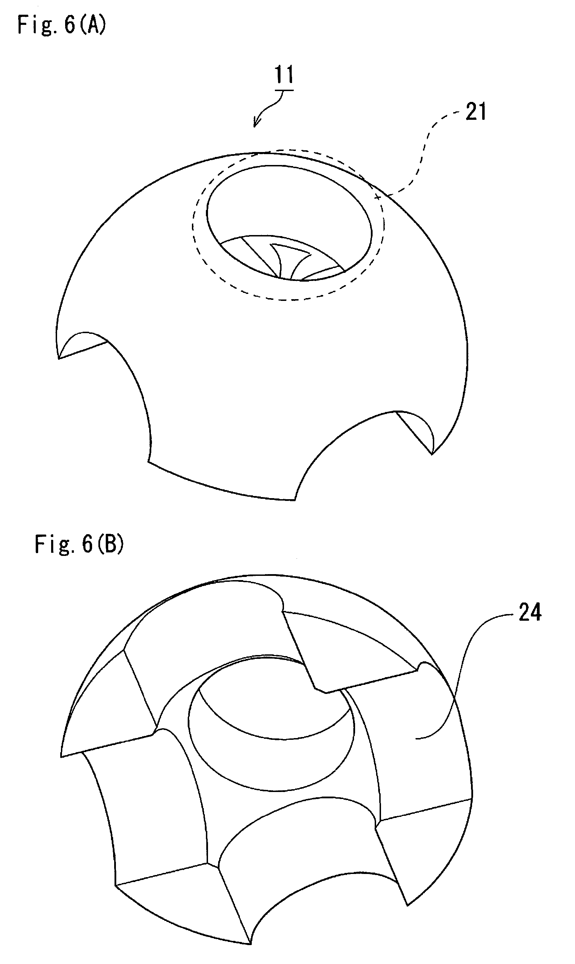

Fig. 6 is a conceptual diagram for explaining an example of a bone filling material having one or a plurality of hollowed parts in the undersurface part. Fig. 6(A) is a perspective view, and Fig. 6(B) is a bottom view. The bone filling material according to this embodiment, for example, has one or a plurality of halved column-shaped hollowed parts in the undersurface part of the hemisphere. It is preferred that the bottom part of hemisphere and the halved surface of the halved column (plane parts which is neither the bottom plane nor the top plane) shares the same plane face. The bone grafting material having these halved hollowed parts (24) can maintain the strength of the bone grafting material and have preferred physical properties for culturing cells and the like. Noted that the bone grafting material described in Fig. 6 can adopt the composition of the bone filling material described in Fig. 5 as appropriate. The example of the radius of the circle which is the halved column-shaped hollowed parts in the undersurface part of the hemisphere is from r/10 to 2r/3, and is preferably from r/5 to r/2.

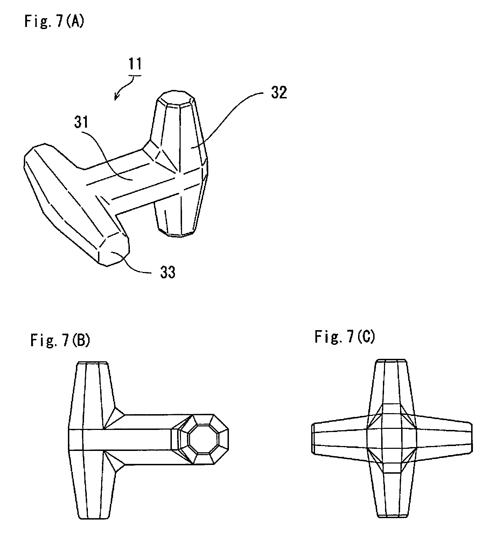

Fig. 7 is a conceptual diagram for explaining an example of a double-headed-shaped bone filling material. Fig. 7(A) is a perspective view, and Fig. 7(B) and Fig. 7(C) are side views. The bone filling material according to this embodiment comprises double-headed parts (32, 33) on both ends of the body part (31). These bone grafting materials shaped like this entwined with each other, thereby improving the strength of the whole of the bone grafting materials as well as making the size of the continuous holes appropriate. Fig. 7 shows an example of a bone filling material whose head part is connected to the body part in T-shape, but the head part may be cross-shaped, furthermore a number of protruding parts may be provided at every predetermined angle. Although, Fig.7 showed an example that the two head parts are shifted by 90 degrees, the angle of the two head parts may be 45 degrees, 0 degree, or 30 degree.

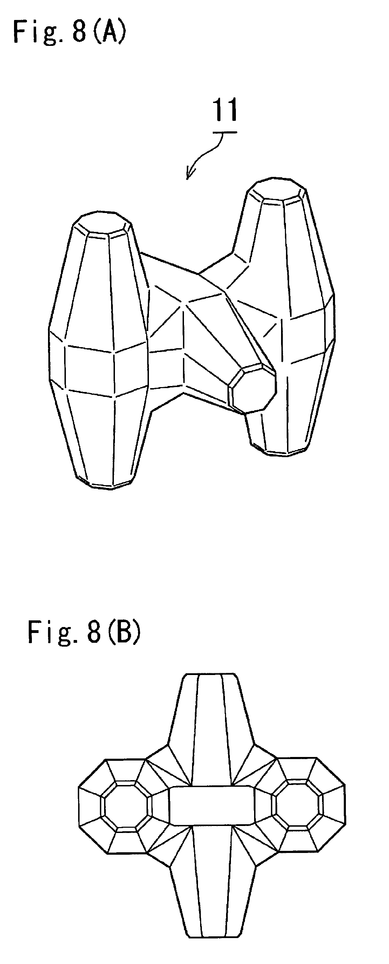

Fig. 8 is a conceptual diagram for explaining an example of a bone filling material having protruding parts on the center thereof. Fig. 8(A) is a perspective view, and Fig. 8(B) is a side view. The bone filling material according to this embodiment is a bone filling material shown in Fig. 7 further comprising protruding parts which penetrate through the body part (31). These bone grafting materials shaped like this entwined with each other, thereby improving the strength of the whole of the bone grafting materials as well as making the size of the continuous holes appropriate. The bone filling material according to this embodiment can adopt the composition of the bone filling material described in Fig.7 as appropriate.

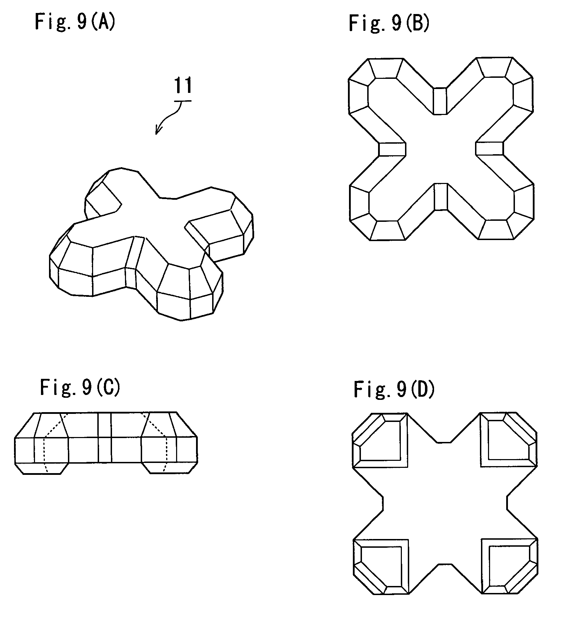

Fig. 9 is a conceptual diagram for explaining an example of a cross-shaped bone filling material. Fig. 9(A) is a perspective view, Fig. 9(B) is a top view, Fig. 9(C) is a side view, and Fig. 9(D) is a bottom view. It is preferred that the bone filling material according to this embodiment, as shown in Fig. 9(C) and Fig. 9(D), has foot part at each end part of the bottom of the cross. The bone grafting material having this shape can make the size of the continuous holes appropriate.

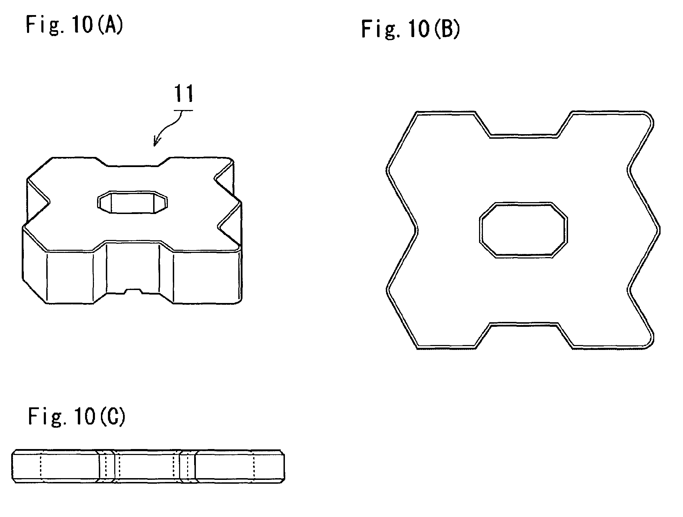

Fig. 10 is a conceptual diagram for explaining an example of a nearly planar bone filling material. Fig. 10(A) is a perspective view, Fig. 10(B) is a top view, and Fig. 10(C) is a side view. The bone filling material according to this embodiment has a hole part in the center of the bone filling material and groove parts on the side surfaces. The bone grafting material having this shape can make the size of continuous holes appropriate.

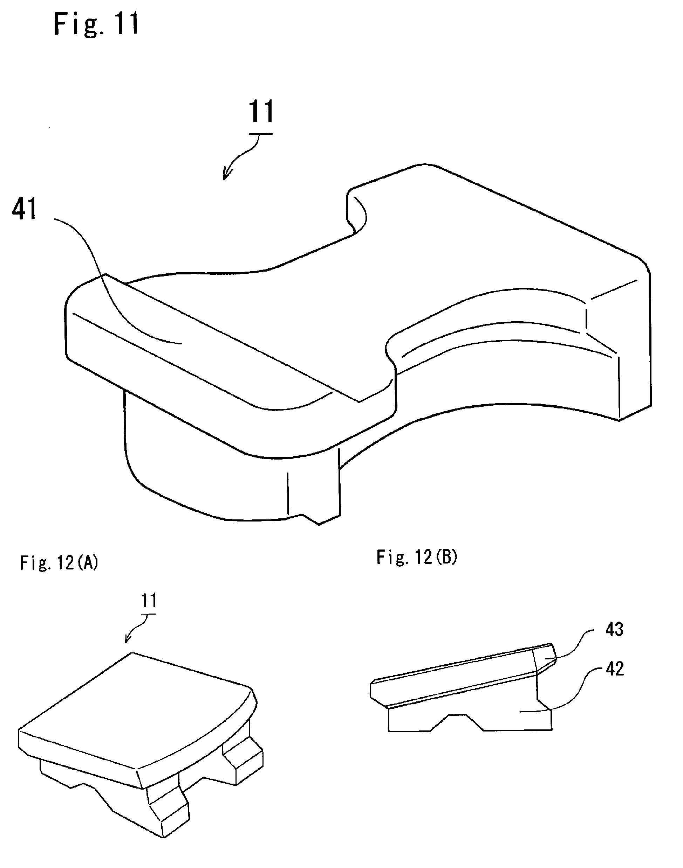

Fig. 11 is a conceptual diagram for explaining an example of a bone filling material having protruding parts on one or both ends of the plane face. Since the bone filling material according to this embodiment has a protruding part on the edge part of a flat body part, it cannot simply be piled up. Also, the bone filling material according to this embodiment preferably has dent portions in the body part as shown in Fig. 11. The bone grafting material having this shape can make the size of continuous holes appropriate.

Fig. 12 is a conceptual diagram for explaining an example of a bone filling material having an inclined top surface. Fig. 12(A) is a perspective view, and Fig. 12(B) is a side view. As shown in Fig. 12, the bone filling material according to this embodiment comprises a foot part (42), and a table part (43) mounted on a foot part (42). And the bottom face of the foot part (42) and the top face of the table part (43) have inclined structures. The bone grafting material according to this embodiment having this shape (inclined top face) cannot simply be piled up, which ensures the existence of continuous holes.

### Usage of Bone Filling Material

The bone filling material produced by the production method of the present invention is injected in bone defect sites, osteoporosis sites, or bone elongation sites. Also, in addition to be filled in the gaps of bones such as bone defect sites, it can also be used as a predetermined carrier of pharmaceutical agents. In this way, the bone filling material can be used for treating or preventing not only bone related diseases but also various other diseases.

### Three-Dimensional Cell Culture Carrier Used In Vitro

Next, a three-dimensional cell culture carrier according to a preferred aspect of the present invention is explained. The three-dimensional cell culture carrier basically uses the above described bone filling material as needed. Namely, the three-dimensional cell culture carrier of the present invention has a plurality of protruding parts, and includes a bone filling material containing calcium-based material. As demonstrated in the example described below, the bone filling material of the present invention can culture cells three-dimensionally, because the bone filling material has preferred voids through which cultured cells can develop. Namely, the present invention can provide an in vitro cell culture system which is an administration of the bone filling material.

It is preferred that the three-dimensional cell culture carrier use a plurality of the above described bone filling materials. The height of the three-dimensional cell culture carrier is adjusted in accordance with the amount of cells to be obtained. The example of the range of the height is from 1 µm to 1 m, preferably from 3 µm to 10 cm, more preferably from 10 µm to 5 cm, and further preferably from 50 µm to 1 cm.

Cells can develop through porous bone filling material effectively. So, it is preferred that the bone filling material, for example, include the above described salts as appropriate. A porous bone filling material can be obtained by immersing the obtained sintering body (which is obtained by sintering the mixture of ingredient and salt component such as sodium chloride) in water and dissolving salts. Also, when minute cracks are made by lowering sinterability, cells can be taken inside the bone filling material, which enables cell culture preferably. Sinterability is lowered, for example, by lowering the sintering temperature or using larger-grained ingredient powders.

A preferred embodiment of the three-dimensional cell culture carrier of the present invention is one wherein an adhesiveness-imparting agent is impregnated or administered on the surface of the bone filling material. Since the above described three-dimensional cell culture carrier maintains a preferred steric structure, cell culture can be continued effectively. The adhesiveness-imparting agent above explained can be used for the three-dimensional cell culture carrier in the same manner. The cultured cells may be administered in vivo. Since the above described adhesiveness-imparting agent has excellent biocompatibility, the cultured cells can be administered in vivo with little risk to the human body.

A usage example of a bone filling material according to a certain aspect of the present invention is a separating carrier for chromatography comprising bone filling material including a plurality of protruding parts and containing calcium-based material. As demonstrated in the example described below, the bone filling material of the present invention has preferred sorbability, and the sorbability can be controlled, for example, by adjusting voids of the bone filling material. So a plurality of bone filling materials can be used as separating carriers for chromatography. A specific usage of chromatography is as follows. Proper amount of bone filling material is filled in a column for chromatography, and solution is injected in the column.

### Example 1

Hereinafter, a method for producing the bone filling material of the present invention is explained specifically using an example. However the present invention is not specifically limited to the example, and includes modifications, which those skilled in the art can think of.

### 1. Kneading Step

α-TCP (produced by Taihei Chemical Industrial, grain size 10µm) was used as powder ingredient. Binder was contained in amounts of 24 parts by weight with respect to 100 parts by weight of ingredient powder. Ethylene-vinyl acetate copolymer, polybutylmethacrylate, paraffin wax, dibutylphthalate and stearic acid were mixed to produce binder. The blending ratio (weight ratio) thereof was 30:30:30:5:5. A 300cc pressure type kneader was heated to 150°C, and binder was poured in the kneader in the order from the binder having the highest melting point to the binder having the lowest melting point. And then the mixture was kneaded for 60 minutes before cooling down. The resultant kneaded material was crashed in a pot mill made of ceramics, and was used as material (a compound or pellet) for molding.

### 2. Molding Step

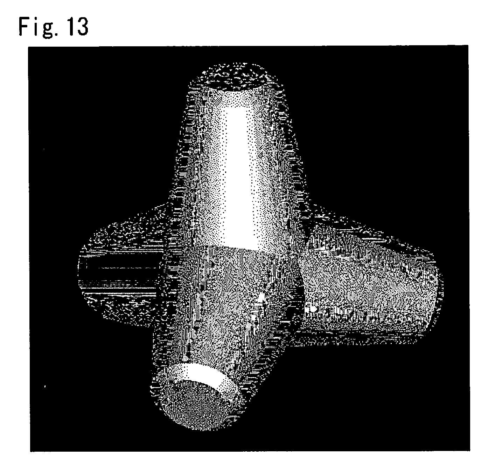

A mold was manufactured based on a CAD image of the bone filling material. Fig. 13 is a CAD generated drawing of a bone filling material. A twelve-cavity mold made of SKD11 was used. The mold has four protruding parts. A gate intake is provided on one of them, and mold parting face is provided along the center line of the remaining three protruding parts between the fixed side and the movable side as deformed part. Ejector pins are arranged inside the three protruding parts. A horizontal injection molding machine (mold locking force: 12 tons) was used. The initial set value of the injection pressure was set at 12GPa. The temperature of the cylinder of the molding machine was set at 130°C, and the temperature of the mold was set at 20°C.

### 3. Binder Removal Step

Molded body was heated to 1000°C (maximum temperature) from ambient atmosphere in an atmosphere degreasing furnace, and the temperature was maintained for an hour before cooling down. The binder removal step lasted 18 hours including cooling down period. A setter made of 90% of alumina (porosity 20%) was used.

### 4. Sintering Step

A degreased body was heated to 1000°C (maximum temperature) from ambient atmosphere, and then the temperature was maintained for an hour before cooling down. The sintering step lasted 18 hours including cooling down period. The setter which was used in the binder removal step was used. The flexural strength of the resultant bone filling material was 6.1MPa (n =18).

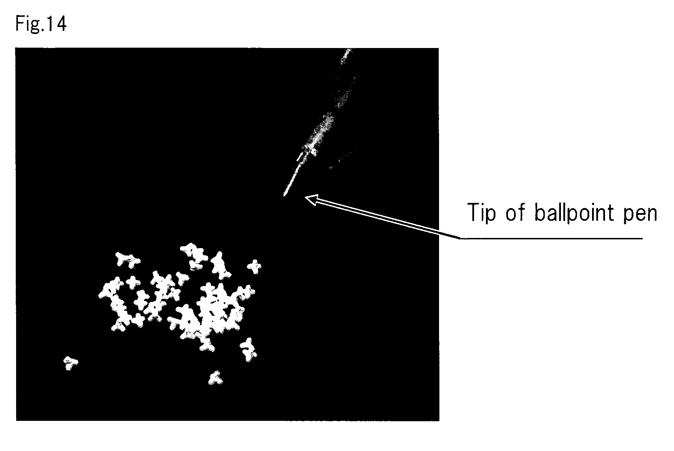

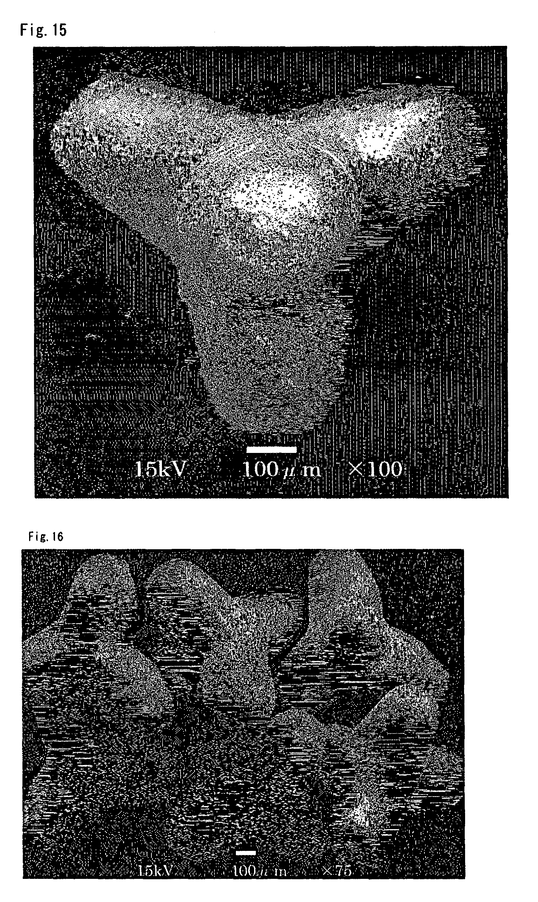

The resultant bone filling material is shown in Fig. 14 to 16. Fig. 14 is a photograph, in place of a diagram, showing a bone filling material obtained in Example 1. Fig. 15 is an electron microgram, in place of a diagram, showing a bone filling material obtained in Example 1. Fig. 16 is an electron microgram, in place of a diagram, showing integrated bone filling materials obtained in Example 1.

### Test Example 1

### Verification of Sintering Temperature of the Bone Filling Material and Bending Strength Thereof

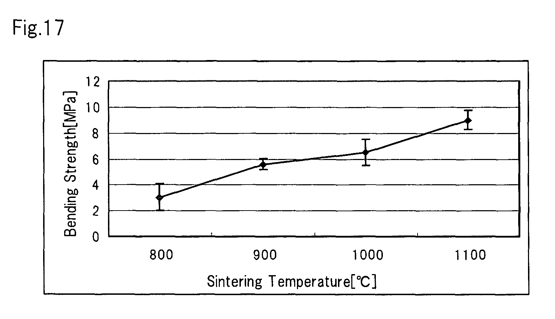

Several kinds of bone filling materials were produced by changing the sintering temperature. And the bending strength of the resultant bone filling material was verified in accordance with JIS R1601. The result is shown in Fig. 17. Fig. 17 shows a relationship between the sintering temperature and the bending strength of the bone filling material obtained in Example 1. It can be seen form Fig. 17 that the bending strength of the bone filling material obtained will be higher, as the sintering temperature thereof becomes higher. It can also be seen that the bending strength of the resultant bone filling material can be controlled by adjusting the sintering temperature. It can further be seen that a bone filling material having bending strength of 2MPa to 10MPa (preferably 4MPa to 9MPa, further preferably 6MPa to 9MPa) can be obtained.

### Test Example 2

### Verification of Filling Condition of the Bone Filling Material In Vivo

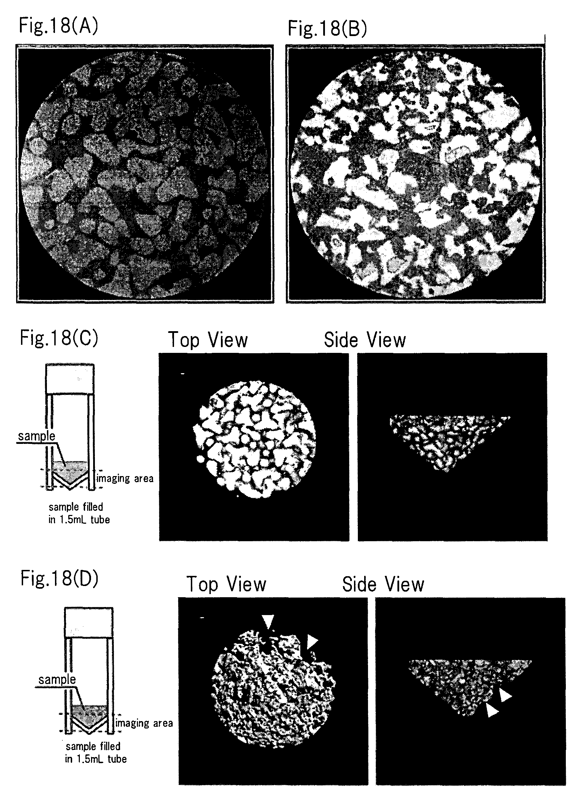

A plurality of bone filling materials form appropriate continuous holes. And bone regeneration is promoted by biological cells penetrating into the continuous holes. So it is preferred that the bone filling material form appropriate continuous holes when it is administered in vivo. In this test example, verification was made on how the bone filling materials of the present invention get together. For comparison, it was also verified whether the existing artificial bone granulation were assembled. The artificial bone granulation were produced by crushing block-shaped bone filling material (OSferion produced by Olympus Co., Japan). In specific, the structure of continuous holes was confirmed by a micro X-ray CT when the bone filling material of the present invention and the existing artificial bone granulation were accumulated in a 2 ml cryotube. The results are shown in Fig. 18. Fig. 18 are CT images by microX-rays, in place of a diagram, showing gatherings of the bone filling materials obtained in Example 1 and the existing artificial bone product. Fig. 18(A) shows gatherings of the bone filling materials of the present invention. Fig. 18(B) shows gatherings of the conventional artificial bone granulation. Fig. 18(C) shows the bone filling materials of the present invention filled in a cryotube. Fig. 18(D) shows the existing bone prosthesis products filled in a cryotube.

It can be seen from Fig. 18 (A) that the bone filling materials of the present invention tend to be self-organizingly accumulated on the joint portion of the pods collectively. As a result, the accumulated bone filling material prevents them from moving relative to each other. It can also be seen that the above described structure of the continuous holes form preferred continuous holes. Since the structure of the continuous holes reflects the length of the pods of the bone filling material and the angle thereof, the structure can be adjusted by changing the length and the angle of the pods. So it can be concluded that the bone filling material of the present invention has excellent morphologic stability and build bone developing area as appropriate. Furthermore, the CT images showed that the density of the bone filling material was almost uniform.

On the other hand, as shown in Fig. 18(B), it is considered that the artificial bone granulation has uneven inter-grain distance which causes problems in reproducibility and morphologic stability of the structure of the continuous holes. In particular, when artificial bone granulations are embedded in a wounded area which is rich in biological fluid, it is highly likely that the inter-grain structure thereof collapses. So it is conceivable that therapeutic effects can not be expected by using the artificial bone granulations. The artificial bone granulations have air bubbles of several hundred micron diameter which are contained in the granulations. When the artificial bone granulations are administered in vivo, cells are likely to be penetrated into the air bubbles of the granulations. But the shape of the air bubbles is uneven, which is not always a preferred shape for promoting osteogenesis. From this perspective, it can also be considered that a desirable osteogenesis effect can not be obtained by using the artificial bone granulations. Also, the density of the existing artificial bone granulations is uneven having low density parts.

It can be seen from Fig. 18(C) that the bone filling material of the present invention has excellent filling property, which makes the material filled along the wall of the cryotube. In contrast, it can be seen from Fig. 18(D) that the existing artificial bone products are not filled along the wall of the cryotube, which may cause voids. (see the arrow of Fig. 18(D)). These voids cause a collapse of homeostasis when the artificial bone and the like are embedded. Namely, it is desirable that proper sized holes or continuous holes are formed when a plurality of filling materials are get together, but it is not desirable that too large holes, such as voids, are formed. Thus, it can be seen that the bone filling material of the present invention act as preferred bone filling material, when a plurality of them are administered.

### Test Example 3

### Verification of the Bone Filling Material as Three Dimensional Cell Culture

Osteoblast-like cell line MC3T3 (MC3T3-E1 (mouse calvarium-derived osteoblast-like cell lines)) was plane cultured, and when it became confluent, the bone filling material unit obtained in Example 1 was placed thereon. The cell culture was continued, and the unit was observed with a microscope over time. The culture medium was a Dullbeco's Eagle's modified medium (DEM media) wherein 10% FBS and 1% penicillin - streptomycin was added. The medium was replaced once every four days. To make a comparison, an observation was made on bone filling material which was set on a media wherein the cell line was not added. Alkaline Phosphatase (ALP) was stained on the osteoblasts in six days and ten days. It was confirmed that the bone filling material had a preferred cellular adhesiveness. The results are shown in Fig. 19.

Fig. 19(A) to (B) are photographs, in place of diagrams, examining cellular adhesiveness of a bone filling material of the present invention. Fig. 19 (A) is a photograph showing a bone filling material without cultured cells after four days culture. Fig. 19 (B) is a photograph, after four days culture, showing a bone filling material on which osteoblast-like cell lines MC3T3 were cultured. Fig. 19 (C) is a photograph showing a bone filling material without cultured cells which is Alkaline Phosphatase (ALP) stained after six days culture. Fig. 19 (D) is a photograph, Alkaline Phosphatase (ALP) stained after six days culture, showing a bone filling material on which osteoblast-like cell lines MC3T3 were cultured. Fig. 19 (E) is a photograph showing a bone filling material without cultured cells which is Alkaline Phosphatase (ALP) stained after ten days culture. Fig. 19 (F) is a photograph, Alkaline Phosphatase (ALP) stained after ten days culture, showing a bone filling material on which osteoblast-like cell lines MC3T3 were cultured.

It can be seen from Fig. 19(A) and 19(B) that there are no cells attached on the surface of the bone filling material set on the medium without cell lines, but there are cells attached on the surface of the bone filling material set on the medium with cell lines. The photograph showing Alkaline Phosphatase (ALP) stained bone filling material after six days culture (shown in Fig. 19(D)) shows how the osteoblasts climb the side gradient of the bone filling material. It can be seen from Fig. 19(F) that there are cells attached on all over the surface of the bone filling material which was cultured for ten days. Therefore, it can also be seen that the bone filling material of the present invention acts effectively as a three-dimensional culture carrier.

The bone filling material produced by the production method of the present invention is injected in bone defect sites, osteoporosis sites, or bone elongation sites. Also, in addition to be filled in the gaps of bones such as bone defect sites, it can also be used as a predetermined carrier of pharmaceutical agents. So the bone filling material of the present invention can be used in the field of pharmaceutical industry and the like.

Cell culture has been so far performed two-dimensionally. But, in contrast, the present invention can provide a carrier which can perform cell culture three-dimensionally. So it can be used in the field of pharmaceutical and biotechnology industries in which cell culture is performed.

## Claims

1. A method for producing a bone filling material, comprising the steps of:
(a) kneading ingredient comprising calcium-based material and material comprising binder;
(b) molding a molded body having a predetermined shape from a kneaded material obtained in step (a) with an injection molding machine having a mold;
(c) removing the binder contained in the molded body (i.e., degreasing) to obtain a degreased body, the molded body being obtained in step (b); and
(d) heating and sintering the degreased body to obtain a sintered body, the degreased body being obtained in step (c).

2. The method for producing a bone filling material as claimed in claim 1,
wherein the calcium-based material comprises one or both of calcium phosphate-based material and calcium carbonate-based material.

3. The method for producing a bone filling material as claimed in claim 1,
wherein the calcium based-material comprises calcium phosphate-based material, and
wherein the calcium phosphate-based material is one or more than one kind of hydroxyl apatite, carbonate apatite, fluorapatite, chlorapatite, β-TCP, α-TCP, calcium metaphosphate, tetra calcium phosphate, calcium hydrogen phosphate, octa calcium phosphate, calcium dihydrogen phosphate, calcium pyrophosphate, the salts thereof, and the solvates thereof.

4. The method for producing a bone filling material as claimed in claim 1,
wherein the binder comprises (meta) acrylic-based resin, wax lubricant, and lubricant.

5. The method for producing a bone filling material as claimed in claim 1, wherein the binder comprises wax lubricant, and
wherein the wax lubricant comprises wax having melting point of 40°C to 100°C.

6. The method for producing a bone filling material as claimed in claim 1,
wherein the binder comprises (meta) acrylic-based resin, ethylene-vinyl acetate copolymer, paraffin wax, stearic acid and dibutyl phthalate, and
wherein the step (a) comprises the steps of:
putting the (meta) acrylic-based resin and the ethylene-vinyl acetate copolymer in a kneading machine;
putting the ingredient, the paraffin wax, and the stearic acid in the kneading machine while kneading the (meta) acrylic-based resin and the ethylene-vinyl acetate copolymer; and
putting the dibutyl phthalate in the kneading machine while kneading the (meta) acrylic-based resin, the ethylene-vinyl acetate copolymer, the paraffin wax, and the stearic acid.

7. The method for producing a bone filling material as claimed in claim 1, the material comprising binder further comprising glass components.

8. The method for producing a bone filling material as claimed in claim 1, the material comprising binder further comprising salt or sugar.

9. The method for producing a bone filling material as claimed in claim 1,
wherein the bone filling material has a plurality of protruding parts, and
wherein the mold comprises:
a fixed mold having an inlet where material is injected;
a movable mold being contacted with the fixed mold when the material is injected, the movable mold being apart from the fixed mold after a molded body is formed,
wherein the inlet of the fixed mold is located at a tip part of one of the protruding parts so that the material for injection molding is injected from the tip part of the protruding part,
wherein a parting face of the fixed mold and the movable mold has an inclined surface, the inclined surface inclines toward the inlet of the fixed mold from the edge face to the center of the parting face, and
wherein the movable mold has dent parts for providing predetermined wedges to the movable mold.

10. The method for producing a bone filling material as claimed in claim 9, wherein the height of the wedge is 1 µm to 1x10² µm.

11. The method for producing a bone filling material as claimed in claim 9,
wherein the movable mold has one or a plurality of grooves with the depth of 1 µm to 2x10 µm.

12. The method for producing a bone filling material as claimed in claim 9, wherein the bone filling material has four protruding parts, and wherein the movable mold has an ejector pin located inside the protruding part.

13. The method for producing a bone filling material as claimed in claim 1, wherein the bone filling material has a plurality of protruding parts, and wherein the mold comprises:
a fixed mold;
a movable mold being contacted with the fixed mold when material is injected, the movable mold being apart from the fixed mold after a molded body is formed,
wherein an inlet of the material for injection molding is located at a parting face of the fixed mold and the movable mold,
wherein the parting face of the fixed mold and the movable mold has an inclined surface, the inclined surface inclines toward the center of the bone filling material from the edge face to the center of the parting face, and
wherein the movable mold has groove parts for providing predetermined wedges to the movable mold.

14. The method for producing a bone filling material as claimed in claim 1,
wherein the step (c) comprises a step of heating-up at 1 °C/hour to 3x10² °C/hour until the temperature reaches a value in the range of 110 °C to 300 °C, which is the range of a first maintaining period.

15. The method for producing a bone filling material as claimed in claim 1, further comprising the step of impregnating or administering a pharmaceutical agent to the sintered body obtained in step (d).

16. The method for producing a bone filling material as claimed in claim 1, further comprising the step of impregnating or administering a pharmaceutical agent to the sintered body obtained in step (d),
whererin the pharmaceutical agent comprises one or more than one kind of an osteogenesis/chondrogenesis promoter (including chondrogenesis promoting factor), a joint disease therapeutic agent, a preventive and/or therapeutic agent for bone/cartilage disease, a bone-regenerating agent, a bone resorption-suppressing substance, an angiogenesis promoter, an antibacterial agent, an antibiotics, or an anticancer agent.

17. The method for producing a bone filling material as claimed in claim 1, further comprising the step of impregnating or administering an adhesiveness-imparting agent to the sintered body obtained in step (d).

18. The method for producing a bone filling material as claimed in claim 1, further comprising the step of impregnating or administering an adhesiveness-imparting agent to the sintered body obtained in step (d),
wherein the adhesiveness-imparting agent is thrombin.

19. The method for producing a bone filling material as claimed in claim 1, further comprising the step of:
preparing a composition including two kinds of adhesiveness-imparting agents;
obtaining a first bone filling material group by impregnating a certain bone filling material group with a first composition or by administering a first composition to a certain bone filling material group;
obtaining a second bone filling material group by impregnating a certain bone filling material group with a second composition or by administering a second composition to a certain bone filling material group,
wherein the bone filling material groups including the first bone filling material group and the second bone filling material group are used as the bone filling materials, and
wherein the first composition comprises:
one or more than one kind of compound represented by the below-described general formula (I) or (II), or a first compound including 3 to 8 repeating units represented by the below-described general formula (III); and
a first diluent (or a carrier), and
wherein the second composition comprises:
one or more than one kind of compound represented by the below-described general formula (I) or (II), or a second compound including 3 to 8 repeating units represented by the below-described general formula (III); and
a second diluent:
X₁ - (OCH₂CH₂)ₙ - X₂ (I)
wherein X₁ and X₂ are the same or different, and each represents -R¹COONHS (where R¹ is a C₁₋₇ alkylene group), -COR¹COONHS (where R¹ is a C₁₋₇ alkylene group), -NOCOR¹-R² (where R¹ is a C₁₋₇ alkylene group, and R² is a maleimide group), -R¹NH₂ (where R¹ is a C₁₋₇ alkylene group), -R¹SH (where R¹ is a C₁₋₇ alkylene group), or -CO₂PhNO₂ (where Ph is an o-, m-, or p-phenylene group), and n is an integer of 80 to 1000; 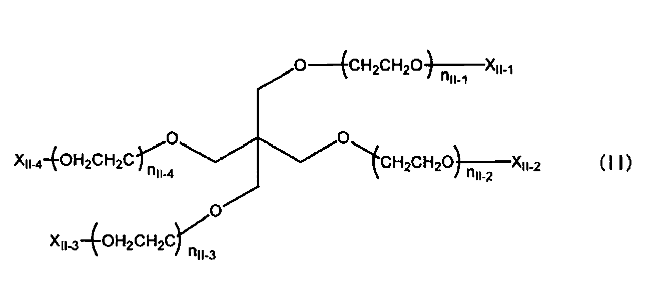
wherein X_{II-1} to X_{II-4} are the same or different, and each represents -R¹COONHS (where R¹ is a C₁₋₇ alkylene group), -COR¹COONHS (where R¹ is a C₁₋₇ alkylene group), -NOCOR¹-R² (where R¹ is a C₁₋₇ alkylene group, and R² is a maleimide group), -R¹NH₂ (where R¹ is a C₁₋₇ alkylene group), -R¹SH (where R¹ is a C₁₋₇ alkylene group), or -CO₂PhNO₂ (where Ph is an o-, m-, or p-phenylene group), and n_{II-1}, to n_{II-4} are the same or different and each represents an integer of 20 to 250; 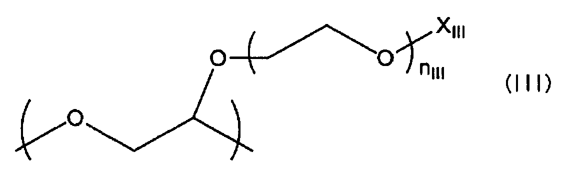
wherein X_{III} represents -R¹COONHS (where R¹ is a C₁₋₇ alkylene group), -COR¹COONHS, -NOCOR¹-R² (where R¹ is a C₁₋₇ alkylene group, and R² is a maleimide group), -R¹NH₂ (where R¹ is a C₁₋₇ alkylene group), -R¹SH (where R¹ is a C₁₋₇ alkylene group), or -CO₂PhNO₂ (where Ph is an o-, m-, or p-phenylene group), and n_{III} represents an integer of 10 to 150.

20. The method for producing a bone filling material as claimed in claim 19,
wherein the first compound comprises one or more than one kind of compound represented by the general formula (I) or (II),
wherein X₁, X₂ or X_{II-1} to X_{II-4} are the same or different, and each represents -NOCOR¹-R² or -R¹NH₂, and
wherein the second compound comprises one or more than one kind of compound represented by the general formula (I) or (II),
wherein X₁, X₂ or X_{II-1} to X_{II-4} are the same or different, and each represents -COR¹COONHS, -R¹SH, or -CO₂PhNO₂ (where Ph is an o-, m-, or p-phenylene group).

21. The method for producing a bone filling material as claimed in claim 1,
wherein the bone filling material has four protruding parts, the four protruding parts extending from the center of the regular tetrahedron form of the bone filling material toward each vertex thereof.

22. A bone filling material comprising:
a plurality of protruding parts, wherein a pharmaceutical agent is impregnated or administered on the surface of the protruding parts; and
calcium-based materials.

23. A bone filling material comprising:
a plurality of protruding parts, wherein an adhesiveness-imparting agent is impregnated, administered or powder blended on the surface of the protruding parts; and
calcium-based materials.

24. A three-dimensional cell culture carrier comprising:
a plurality of protruding parts; and
a bone filling material including calcium-based materials.

25. The three-dimensional cell culture carrier as claimed in claim 24,
wherein a pharmaceutical agent or an adhesiveness-imparting agent is impregnated, administered or powder blended on the surface of the bone filling material.

26. A separating carrier for chromatography comprising:
a plurality of protruding parts; and
a bone filling material including calcium-based materials.
